# EUROPEAN PATENT SPECIFICATION

(11) **EP 3 654 886 B1**
(45) Date of publication and mention of the grant of the patent: **17.07.2024**
(21) Application number: 18756372.1
(22) Date of filing: 20.07.2018
(51) Int. Cl.: A61F 2/24

(54) **A STENTED VALVE**
GESTENTETE KLAPPE
VALVULE À STENT

(30) Priority: 20.07.2017 EP 17182461
(43) Date of publication of application: 27.05.2020
(73) Proprietor: The Provost, Fellows, Foundation Scholars, and The Other Members of Board, of The College of The Holy and Undivided Trinity of Queen Elizabeth, Dublin 2 (IE); National University of Ireland Galway, Galway (IE)
(72) Inventor: MURPHY, Bruce, Dublin (IE); CROWLEY, James, Oranmore (IE); BURKE, Michael, Dublin (IE)
(74) Representative: FRKelly
(86) International application number: PCT/EP2018/069757
(87) International publication number: WO 2019/016366

(56) References cited:
- WO-A2-2008/103497
- US-A1- 2011 137 397
- US-A1- 2016 324 633

## Description

### Field of the invention

The present invention relates to a fixation element for fastening a stented valve that can be used to repair and/or replace dysfunctional heart valves. In particular, the present invention relates to a fixation element for fastening a stented valve that can be used to repair and/or replace a dysfunctional mitral or tricuspid heart valve.

### Background of the invention

When the four valves of a heart are operating in a healthy manner, there is substantial unidirectional blood flow through the chambers of the heart. However, if a heart valve becomes dysfunctional, there is a disruption to the substantial unidirectional blood flow. In the dysfunctional case, blood can flow in the wrong direction through a heart valve, reducing the efficiency of the heart - this irregular blood flow is generally referred to as regurgitation. Depending on the severity of a heart valve's regurgitation, a patient may develop heart failure, and subsequently this may lead to severe health problems.

In the case of a mitral valve, regurgitation can be caused by a number of disease states. In one such disease state, the annulus of the mitral valve enlarges due to enlargement of the left ventricle. The resulting effect of annular enlargement is that the mitral valve's leaflets do not seal in a closed position - this results in a leaky valve when pressure in the left ventricle rises above the left atrial pressure. In another disease state, the chordae tendineae can fracture - this causes the leaflets of the valve to prolapse into the left atrium and, as a consequence, the leaflets do not seal at peak contraction pressures. Further disease states of the mitral valve can include: leaflet perforation, calcification of the leaflets and/or annulus and/or chordae tendineae, and leaflet tethering caused by left ventricle remodelling. In other disease states, such as Barlow's disease, the leaflets of the mitral valve bulge into the left atrium and can cause abnormal behaviour of the mitral valve. In all of these disease states, repair and/or replacement may be required in order to eliminate substantial mitral valve regurgitation. Disease states associated with the tricuspid valve on the right hand side mirror the majority of these disease states, with annular enlargement being the most frequently observed disease state in patients.

Current treatment options include medical therapy, such as diuretics and/or vasodilators, which aim to reduce the amount of blood flowing back into the left atrium. However, medical therapy does not tackle the root cause of mitral or tricuspid valve regurgitation. Other treatment options include surgical, open-heart repair or replacement strategies. In the case of repair strategies, a surgeon will perform a mitral or tricuspid valve reconstruction procedure with or without an annuloplasty ring. In the case of mitral valve replacement, a surgeon will replace the valve with either a mechanical valve or a biological valve. Other surgical repair techniques include suturing the two leaflets of the valve together and implanting an annuloplasty ring - these are invasive procedures that carry a high degree of risk for a patient.

To reduce the risk associated with open heart surgical mitral or tricuspid valve repair procedures, a number of minimally invasive tools and techniques have been developed. Existing systems require a high degree skill for successful use and can result in excessively long procedure times. Progress in the field of minimally invasive aortic valve replacement has demonstrated that a dysfunctional heart valve can be successfully replaced via a minimally invasive manner. Existing devices have demonstrated that, in patients with severe aortic stenosis, who are not suitable candidates for surgery, the rate of death can be significantly reduced in comparison to standard surgical therapy.

In the case of minimally invasive (e.g. catheter-based) replacement of an aortic valve, the target anatomy is relatively simple - the target site is, roughly, a tubular structure. However, the target site for a minimally invasive valve replacement for a mitral or tricuspid heart valve is composed of an irregular geometry rather than a tubular structure. The valve opening is not a circular structure, the valve annulus does not lie in one plane, the valve annulus moves a relatively large amount in comparison to other reference points within a heart, and there are multiple components of the ventricle and valve to be accounted for. Furthermore, the disease states are more diverse in comparison to the disease states associated with the aortic valve.

To account for the multiple geometric challenges and varied disease states associated with the mitral valve, designers and inventors of catheter-based mitral valve replacement devices have adopted multiple, considerably different solutions to replace the mitral valve, utilising catheter-based delivery mechanisms. Key to a design of a transcatheter mitral valve is the mechanism that is utilised to fix the implant at the target site. At present, multiple different approaches have been adopted. For example, several mitral prosthesis are available that utilise a tethering or anchoring system. Other investigators in the field have taken a different approach and propose to implant a docking station, which is separate to the replacement valve. Subsequent to the delivery of a docking station, a replacement valve can be implanted within the regular geometry of the docking station. At present, there is no consensus on the optimal fixation method for a transcatheter mitral valve replacement.

Similar geometric challenges occur in the case of the tricuspid valve and, similarly, the fixation of catheter-based tricuspid valve replacement technologies will require a suitable mechanism to ensure successful device fixation.

US2016324633 discloses an apparatus for use at a native heart valve of a subject. For some applications, the apparatus comprises a prosthetic valve support, configured to be implanted at the native valve, to facilitate at a first period implantation at the native valve of a first prosthetic valve, and to facilitate at a second period implantation at the native valve of a second prosthetic valve.

Given the unique mitral (or tricuspid) valve geometry and associated multiple variable disease states, there exists the need to develop a flexible, minimally-invasive atrioventricular replacement valve, that provides secure fixation at the intended target site.

### Summary of the invention

According to a first aspect of the present invention, there is provided a fixation element for fastening a stented valve to a native heart valve, the fixation element comprising:
(a) a control rod comprising:
   (i) a proximal end, connected to
   (ii) a distal end reversibly connectable to
(b) a pivotable clamp comprising
   (i) at least one arm, wherein the at least one arm comprises a hand, wherein the at least one arm and the hand are located on opposing sides of the pivot of the pivotable clamp; and
   (ii) an actuator operable to displace the pivotable clamp between an open position and a closed position by operation of the proximal end of the control rod.

Optionally, the control rod is capable of transmitting torque from the proximal end to the distal end. Optionally, the control rod is capable of transmitting contact force from the proximal end to the distal end.

Optionally, the control rod comprises resilient material. Optionally, the control rod is formed from resilient material.

Optionally, the control rod is formed from a material flexible about the longitudinal axis of the control rod. Optionally, the control rod is flexible about the longitudinal axis of the control rod.

Optionally, the control rod has axial flexibility.

Optionally, the control rod comprises at least one depression in the surface of the control rod. Optionally, the at least one depression comprises at least one groove. Optionally, the at least one groove is substantially perpendicular to the axis of the control rod. Optionally, the at least one groove is substantially perpendicular to the axis of the control rod, such that the control rod is flexible along the axis of the control rod.

Optionally, the control rod comprises polymer. Optionally, the control rod is formed from polymer. Optionally, the control rod comprises plastic. Optionally, the control rod is formed from plastic.

Optionally, the control rod comprises metal. Optionally, the control rod is formed from metal. Further optionally, the control rod comprises metal alloy. Further optionally, the control rod comprises a metal alloy of nickel and titanium. Optionally or additionally, the control rod comprises a steel alloy, optionally stainless steel. Further optionally or additionally, the control rod comprises a metal alloy of cobalt and chromium.

Optionally, the fixation element further comprises a controller. Optionally, the control rod may interact with the controller. Optionally the proximal end of the control rod may interact with the controller. Optionally, the controller is operable to actuate the pivotable clamp. Optionally, the controller is operable to close the pivotable clamp. Optionally, the controller is operable to open the pivotable clamp. Optionally, the controller is operable to open or close the pivotable clamp.

Optionally, the controller is operable to rotate the control rod about the longitudinal axis of the control rod. Optionally, the controller is operable to reciprocally move the control rod. Optionally, the controller is operable to reciprocally move the control rod parallel to the longitudinal axis of the control rod.

Optionally, the controller is operable to move the control rod parallel to the longitudinal axis of the control rod. Optionally, the controller is operable to reciprocally move the control rod parallel to the longitudinal axis of the control rod.

Optionally, the sleeve may interact with the controller, optionally at the proximal end of the control rod. Optionally, the controller is operable to move the sleeve parallel to the longitudinal axis of the control rod. Optionally, the controller is operable to reciprocally move the sleeve parallel to the longitudinal axis of the control rod.

Optionally, the controller is operable to move the sleeve relative to the control rod.

Optionally, the controller comprises a rod control part and a sleeve control part. Optionally, the rod control part is connected to the sleeve control part.

Optionally, the rod control part may act on the control rod. Optionally, the rod control part is operable to rotate the control rod about the longitudinal axis of the control rod.

Optionally, the sleeve control part may act on the sleeve. Optionally, the sleeve control part is operable to rotate the sleeve about the longitudinal axis of the control rod. Optionally, the sleeve control part is operable to move the sleeve relative to the control rod. Optionally, the sleeve control part is operable to reciprocally move the sleeve relative to the control rod.

Optionally, the controller further comprises a spacer. Optionally, the controller may further comprise a spacer. Optionally, the rod control part may be connected to the sleeve control part by the spacer. Optionally, the spacer may be displaced such that the rod control part may contact the sleeve control part.

Optionally, the fixation element further comprises a string. Optionally, the string is connected to the pivotable clamp. Optionally, the string is reversibly connectable to the pivotable clamp. Optionally, the string is reversibly connectable to the control rod. Optionally, the string is reversibly connectable to the proximal end of the control rod.

Optionally, the string may interact with the controller. Optionally, the controller is operable to actuate the pivotable clamp via the string. Optionally, the controller is operable to close the pivotable clamp via the string. Optionally, the controller is operable to open the pivotable clamp via the string. Optionally, the controller is operable to open or close the pivotable clamp via the string.

Optionally, the string and control rod may interact with the controller. Optionally, the controller is operable to actuate the pivotable clamp via the string and control rod. Optionally, the controller is operable to close the pivotable clamp via the string and control rod. Optionally, the controller is operable to open the pivotable clamp via the string and control rod. Optionally, the controller is operable to open or close the pivotable clamp via the string and control rod.

Optionally, the fixation element further comprises a sleeve having a sleeve lumen. Optionally, the sleeve is arranged around the control rod. Optionally, the control rod passes through the sleeve lumen. Optionally, the control rod passes through a first section of the length of the sleeve lumen.

Optionally, the string passes through the sleeve lumen. Optionally, the string passes through a second section of the length of the sleeve lumen.

Optionally, there is overlap between the first and second sections of the length of the sleeve lumen.

Optionally, the sleeve extends at least along the length of the control rod, from the proximal end of the control rod to the distal end of the control rod. Alternatively, the sleeve extends along a section of the length of the control rod.

Optionally, the sleeve is moveable relative to the control rod.

Optionally, the distal end of the control rod is reversibly connectable to the pivotable clamp by an interference fit.

Optionally, the distal end of the control rod comprises a key and the pivotable clamp comprises a socket.

Optionally, the key is receivable within the socket. Further optionally, the key is reversibly receivable within the socket. Still further optionally, the key is reversibly receivable within the socket by an interference fit.

Optionally, the distal end of the control rod comprises a hex key and the pivotable clamp comprises a hex socket. Optionally, the hex key is receivable within the hex socket. Further optionally, the hex key is reversibly receivable within the hex socket. Still further optionally, the hex key is reversibly receivable within the hex socket by an interference fit.

Alternatively, the distal end of the control rod comprises a threaded portion and the pivotable clamp comprises a corresponding threaded socket. Optionally, the threaded portion is receivable within the corresponding threaded socket. Further optionally, the threaded portion is reversibly receivable within the corresponding threaded socket.

Optionally, the sleeve covers the distal end of the control rod. Optionally, the sleeve is moveable to cover the distal end of the control rod. Alternatively, the sleeve is moveable to uncover the distal end of the control rod.

Optionally, the sleeve is reversibly movable to cover the distal end of the control rod. Optionally, the sleeve is reversibly movable to uncover at least part of the distal end of the control rod. Optionally, the sleeve is reversibly movable to cover the distal end of the control rod such that a reversible connection between the distal end of the control rod and the pivotable clamp is reversibly coverable by the sleeve.

The pivotable clamp comprises an actuator operable to displace the pivotable clamp between the open position and the closed position. The actuator is operable to displace the pivotable clamp between the closed position and the open position.

Optionally, the distal end of the control rod is reversibly connectable to the actuator.

Optionally, the actuator comprises a reciprocally movable shaft. Further optionally, the actuator comprises a reciprocally movable threaded shaft. Still further optionally, the actuator comprises a reciprocally movable threaded shaft and a correspondingly threaded aperture.

Optionally, the pivotable clamp further comprises a reciprocally movable threaded shaft and a correspondingly threaded aperture, whereby the distal end of the control rod is reversibly connectable to the reciprocally movable threaded shaft.

Optionally, the reciprocally movable shaft is a reciprocally movable threaded shaft.

The actuator acts on the pivotable clamp. Optionally, the reciprocally movable shaft may act on the pivotable clamp.

Optionally, the pivotable clamp comprises at least two arms. Optionally, the pivotable clamp comprises two arms. Still further optionally, the pivotable clamp comprises first and second arms.

Optionally, the reciprocally movable shaft may act on at least one arm of the pivotable clamp.

The at least one arm of the pivotable clamp comprises a hand.

Optionally, the hand is fixed to the at least one arm of the pivotable clamp.

The at least one arm and the hand are located on opposing sides of the pivot of the pivotable clamp.

Optionally, the reciprocally movable shaft may act on the hand of the pivotable clamp. Further optionally, the reciprocally movable shaft may act on the hand of the pivotable clamp to displace the at least one arm between the open position and the closed position. Optionally, the reciprocally movable shaft may act on the hand of the pivotable clamp to displace the at least one arm between the closed position and the open position. Still further optionally, the reciprocally movable shaft may act on the hand of the pivotable clamp to displace the pivotable clamp between the open position and the closed position. Optionally, the reciprocally movable shaft may act on the hand of the pivotable clamp to displace the pivotable clamp between the closed position and the open position.

Optionally, the reciprocally movable shaft may act on the hand of the pivotable clamp to displace the at least one arm about the pivot of the pivotable clamp.

Optionally, the string is connectable to the hand of the pivotable clamp. Optionally, the string is reversibly connectable to the hand of the pivotable clamp. Optionally, the string may act on the hand of the pivotable clamp. Optionally, the string may act on the hand of the pivotable clamp to displace the at least one arm between the open position and the closed position. Optionally, the string may act on the hand of the pivotable clamp to displace the at least one arm between the closed position and the open position. Still further optionally, the string may act on the hand of the pivotable clamp to displace the pivotable clamp between the open position and the closed position. Optionally, the string may act on the hand of the pivotable clamp to displace the pivotable clamp between the closed position and the open position.

Optionally, the distal end of the control rod is reversibly connectable to the actuator. Further optionally, the distal end of the control rod is reversibly connectable to the actuator by an interference fit.

Optionally, the distal end of the control rod comprises a key and the actuator comprises a socket.

Optionally, the key is receivable within the socket. Further optionally, the key is reversibly receivable within the socket. Still further optionally, the key is reversibly receivable within the socket by an interference fit.

Optionally, the distal end of the control rod comprises a hex key and the actuator comprises a hex socket. Optionally, the hex key is receivable within the hex socket. Further optionally, the hex key is reversibly receivable within the hex socket. Still further optionally, the hex key is reversibly receivable within the hex socket by an interference fit.

Optionally, the pivotable clamp is displaceable between the closed position and a locked configuration; whereby operation of the proximal end of the control rod does not displace the pivotable clamp between the locked configuration and the open position.

Optionally, the pivotable clamp is displaceable between the closed position and a locked configuration by operation of the proximal end of the control rod.

Optionally the pivotable clamp further comprises a lock that is operable to displace the pivotable clamp into the locked configuration. Optionally, the actuator may act on the lock. Optionally, the actuator may act on the lock to displace the pivotable clamp between the closed position and the locked configuration.

Optionally, the lock further comprises at least one elastic member. Optionally, the lock further comprises at least one elastic member having an elastic limit.

Optionally, the at least one elastic member may be reversibly deformed from an original position to a deformed position.

Optionally, the at least one elastic member may be reversibly deformed from the original position to the deformed position by displacement of the pivotable clamp from the open position to the closed position. Optionally, the at least one elastic member may be irreversibly deformed from the original position to the deformed position by displacement of the pivotable clamp from the open position to the closed position. Optionally, the at least one elastic member may be irreversibly deformed by operation of the lock. Optionally, the at least one elastic member is irreversibly deformed in the locked configuration of the pivotable clamp.

Optionally, the at least one elastic member may be deformed by movement of the actuator. Optionally, the at least one elastic member may be deformed by movement of the reciprocally movable shaft. Optionally, the at least one elastic member is connected to the hand of the pivotable clamp.

Optionally, the at least one elastic member does not contact the actuator in the open position of the pivotable clamp. Optionally, the at least one elastic member contacts the actuator in the locked configuration of the pivotable clamp. Optionally, the at least one elastic member is substantially parallel to the actuator in the open position of the pivotable clamp.

Optionally, the at least one elastic member does not contact the reciprocally movable shaft in the open position of the pivotable clamp. Optionally, the at least one elastic member contacts the reciprocally movable shaft in the locked configuration of the pivotable clamp. Optionally, the at least one elastic member is substantially parallel to the reciprocally movable shaft in the open position of the pivotable clamp.

Optionally, the at least one elastic member does not contact the reciprocally movable threaded shaft in the open position of the pivotable clamp. Optionally, the at least one elastic member contacts the reciprocally movable threaded shaft in the locked configuration of the pivotable clamp. Optionally, the at least one elastic member is substantially parallel to the reciprocally movable threaded shaft in the open position of the pivotable clamp.

Optionally, in the deformed position the at least one elastic member is in contact with the exterior surface of the pivotable clamp; optionally in the deformed position the at least one elastic member is in slidable contact with the exterior surface of the pivotable clamp.

Optionally, in the deformed position the at least one elastic member is in contact with the hand of the pivotable clamp; optionally in the deformed position the at least one elastic member is in slidable contact with the hand of the pivotable clamp.

Optionally, the at least one elastic member is in the deformed position in the open position of the pivotable clamp. Optionally, the at least one elastic member is in the deformed position in the closed position of the pivotable clamp. Optionally, the at least one elastic member is in the original position in the locked configuration of the pivotable clamp. Optionally, the at least one elastic member is moved by the elasticity of the at least one elastic member to the original position in the locked configuration of the pivotable clamp.

Optionally, the at least one elastic member prevents the pivotable clamp from moving from the locked configuration of the pivotable clamp to the open position of the pivotable clamp.

Optionally, the actuator is detachable, optionally reversibly detachable, from the pivotable clamp. Optionally, the reciprocally movable shaft is detachable, optionally reversibly detachable, from the pivotable clamp; optionally the reciprocally movable threaded shaft is detachable, optionally reversibly detachable, from the pivotable clamp.

Optionally, the at least one elastic member prevents the pivotable clamp from moving from the locked configuration of the pivotable clamp to the open position of the pivotable clamp, in the detached configuration of the actuator. Optionally, the at least one elastic member prevents the pivotable clamp from moving from the locked configuration of the pivotable clamp to the open position of the pivotable clamp, in the detached configuration of the reciprocally movable shaft. Optionally, the at least one elastic member prevents the pivotable clamp from moving from the locked configuration of the pivotable clamp to the open position of the pivotable clamp, in the detached configuration of the reciprocally movable threaded shaft.

Optionally, the control rod comprises the actuator, or reciprocally movable shaft, or reciprocally movable threaded shaft. Optionally, the control rod comprises the actuator, or reciprocally movable shaft, or reciprocally movable threaded shaft. Optionally, the control rod comprises the actuator, or reciprocally movable shaft, or reciprocally movable threaded shaft. Optionally, the distal end of the control rod comprises the actuator, or reciprocally movable shaft, or reciprocally movable threaded shaft.

Optionally, the pivotable clamp comprises an offset hinge. Optionally, the pivot comprises an offset hinge.

Still further optionally, the pivotable clamp comprises a quadrilateral linkage. Still further optionally, the pivotable clamp comprises a crank.

Optionally, the pivotable clamp comprises two arms connected by a pivot. Further optionally, the pivotable clamp further comprises two sub-arms connected by a sub-pivot. Optionally, the pivotable clamp comprises first and second arms connected by a pivot. Further optionally, the pivotable clamp further comprises first and second sub-arms connected by a sub-pivot.

Optionally, the first sub-arm is mountable to the first arm and the second sub-arm is mountable to the second arm. Further optionally, the first sub-arm is fixedly mountable to the first arm and the second sub-arm is pivotably mountable to the second arm.

Optionally, at least one arm of the pivotable clamp further comprises a ball nose tip.

Optionally or additionally, at least one arm of the pivotable clamp further comprises at least one securing means.

Optionally, the at least one securing means is operable between a contracted state and an expanded state.

Optionally, in the contracted state the at least one securing means is substantially coaxial with the longitudinal axis of the at least one arm. Optionally or additionally, in the expanded state, the at least one securing means is substantially perpendicular to the longitudinal axis of the at least one arm.

Optionally, the at least one securing means is formed from a resilient material.

Optionally, the at least one securing means comprises polymer.

Optionally, the at least one securing means comprises metal. Optionally, the at least one securing means is formed from metal. Further optionally, the at least one securing means comprises metal alloy. Further optionally, the at least one securing means is formed from metal alloy. Further optionally, the at least one securing means comprises a metal alloy of nickel and titanium. Further optionally, the at least one securing means is formed from a metal alloy of nickel and titanium.

Optionally, the at least one securing means comprises titanium.

Optionally, the at least one securing means further comprises a surface cover for encouraging fibrotic tissue growth, the surface cover comprising: porous polymeric material. Optionally or additionally, the surface cover comprises porous polymeric plastic. Optionally or additionally, the surface cover comprises polyester. Further optionally or additionally, the surface cover comprises synthetic polyester. Further optionally or additionally, the surface cover comprises a material selected from the group comprising: polyethylene terephthalate (PET), polyethylene terephthalate fabric (PET fabric), polytetrafluoroethylene (PTFE), and expanded polytetrafluoroethylene (ePTFE).

Optionally, operation of the proximal end of the control rod actuates operation of the distal end of the control rod.

Optionally or additionally, operation of the distal end of the control rod actuates operation of the pivotable clamp. Further optionally or additionally, operation of the key of the distal end of the control rod actuates operation of the socket of the pivotable clamp.

Optionally or additionally, operation of socket of the pivotable clamp actuates the actuator to displace the pivotable clamp between the open position and the closed position. Further optionally or additionally, operation of socket of the pivotable clamp actuates the reciprocally movable threaded shaft in the correspondingly threaded aperture.

Optionally or additionally, operation of the reciprocally movable threaded shaft in the correspondingly threaded aperture may act on at least one arm of the pivotable clamp. Further optionally or additionally, operation of the reciprocally movable threaded shaft in the correspondingly threaded aperture may act on the hand of the at least one arm of the pivotable clamp. Still further optionally or additionally, operation of the reciprocally movable threaded shaft in the correspondingly threaded aperture may act on the hand of the pivotable clamp to displace the at least one arm between the open position and the closed position. Still further optionally or additionally, operation of the reciprocally movable threaded shaft in the correspondingly threaded aperture may act on the hand of the pivotable clamp to displace the at least one arm about the pivot of the pivotable clamp.

Also disclosed is a method of manufacture of a fixation element according to present invention. According to a second aspect of the present invention, there is provided a coupling for a stented valve and the fixation element according to the first aspect of the present invention, the coupling comprising:
(a) a connector adapted to receive the fixation element, and
(b) a guide adapted to receive the stented valve;
wherein the connector is reciprocally movable relative to the guide.

Optionally, the connector is slidable relative to the guide. Further optionally, the connector is reciprocally slidable relative to the guide.

Optionally, the connector is insertable into the guide. Optionally, the connector is retractable from the guide. Optionally, the connector and guide have a male-female interaction.

Optionally, the guide is a pocket. Optionally, the connector is insertable into the pocket. Optionally, the connector is retractable from the pocket. Optionally, the connector and pocket have a male-female interaction.

Optionally, the connector is in communication with the guide. Further optionally, the connector is in moveable communication with the guide. Still further optionally, the connector is in slidable communication with the guide.

Optionally, the guide has a first end and a second end; whereby the connector is reciprocally movable between the first end and second end of the guide. Further optionally, the guide has a first end and a second end; whereby the connector is slidable between the first end and second end of the guide. Further optionally, the guide has a first end and a second end; whereby the connector is reciprocally slidable between the first end and second end of the guide.

Optionally, the connector is receivable within the guide. Further optionally, the connector is reversibly receivable within the guide. Alternatively, the connector is irreversibly receivable within the guide.

Optionally, the connector comprises a projection receivable within the guide. Further optionally, the connector comprises a projection reversibly receivable within the guide. Alternatively, the connector comprises a projection irreversibly receivable within the guide.

Optionally, the guide comprises a track. Optionally, the guide comprises a track, and the connector is receivable by the track. Optionally, the guide comprises a track, and the connector comprises a projection receivable by the track.

Alternatively, the guide comprises a channel. Optionally, the guide comprises a channel, and the connector is receivable within the channel. Optionally, the guide comprises a channel, and the connector comprises a projection receivable within the channel.

Alternatively, the guide comprises a slot, and the connector comprises a projection receivable within the guide. Further optionally, the guide comprises a slot, and the connector comprises a projection receivable within the slot.

Optionally, the projection comprises a stop to inhibit the passage of the connector through the guide. Further optionally, the projection comprises a stop to inhibit the passage of the connector through the slot.

Alternatively, the guide comprises a tube having a tube lumen, and the connector is receivable within the guide. Optionally, the guide comprises a tube lumen, and the connector is receivable within the guide. Optionally, the guide comprises a tube lumen, and the connector is receivable within the tube lumen.

Optionally, the tube comprises a slit tube, and the connector is receivable within the slit tube lumen. Optionally, the tube comprises a slit tube, and the connector comprises a projection receivable within the slit tube lumen.

Optionally, the connector is receivable within the guide. Further optionally, the connector is reversibly receivable within the guide. Alternatively, the connector is irreversibly receivable within the guide.

Optionally, the connector is receivable within the tube lumen of the guide. Further optionally, the connector is reversibly receivable within the tube lumen of the guide. Alternatively, the connector is irreversibly receivable within the tube lumen of the guide.

Optionally, the connector is locatable within the tube lumen of the guide. Further optionally, the connector is reversibly locatable within the tube lumen of the guide. Alternatively, the connector is irreversibly locatable within the tube lumen of the guide.

Optionally, the connector is concentrically locatable within the guide. Further optionally, the connector is concentrically reversibly locatable within the guide. Alternatively, the connector is concentrically irreversibly locatable within the guide.

Optionally, the connector is concentrically locatable within the tube lumen. Further optionally, the connector is concentrically reversibly locatable within the tube lumen. Alternatively, the connector is concentrically irreversibly locatable within the tube lumen.

Optionally or additionally, the connector is coaxially locatable within the tube lumen. Further optionally, the connector is coaxially reversibly locatable within the tube lumen. Alternatively, the connector is coaxially irreversibly locatable within the tube lumen.

Optionally, the connector is not rotatable within the guide.

Optionally, at least one of the tube lumen and the connector has a non-circular cross section. Further optionally, each of the tube lumen and the connector has a non-circular cross section.

Optionally, at least one of the tube lumen and the connector has a polygonal cross section. Optionally, at least one of the tube lumen and the connector has a substantially oval cross section.

Also disclosed is a method of manufacture of a coupling according to the first aspect of the present invention.

According to a second aspect of the present invention, there is provided a prosthetic valve comprising a stented valve, and the at least one fixation element according to the first aspect of the present invention, wherein the stented valve and the at least one fixation element are each received by at least one coupling.

Optionally, the prosthetic valve comprises a stented valve, and the at least one fixation element according to the first aspect of the present invention, wherein the stented valve and the at least one fixation element are each received by at least one coupling according to the second aspect of the present invention.

Optionally, the prosthetic valve comprises:
(a) a stented valve;
(b) a coupling comprising:
   (i) a connector adapted to receive the fixation element, and
   (ii) a guide adapted to receive the stented valve;
      wherein the connector is reciprocally movable relative to the guide; and
(c) the fixation element according to the first aspect of the present invention.

Optionally, the prosthetic valve comprises:
(a) a stented valve;
(b) at least one coupling, each coupling comprising:
   (i) a connector adapted to receive the fixation element, and
   (ii) a guide adapted to receive the stented valve;
      wherein the connector is reciprocally movable relative to the guide; and
(c) at least one respective fixation element according to the first aspect of the present invention.

Optionally, the prosthetic valve comprises:
(a) a stented valve;
(b) two couplings, each coupling comprising:
   (i) a connector adapted to receive the fixation element, and
   (ii) a guide adapted to receive the stented valve;
      wherein the connector is reciprocally movable relative to the guide; and
(c) two respective fixation elements according to the first aspect of the present invention.

Optionally, the prosthetic valve comprises:
(a) a stented valve;
(b) three couplings, each coupling comprising:
   (i) a connector adapted to receive the fixation element, and
   (ii) a guide adapted to receive the stented valve;
      wherein the connector is reciprocally movable relative to the guide; and
(c) three respective fixation elements according to the first aspect of the present invention.

Optionally, the prosthetic valve comprises:
(a) a stented valve;
(b) a plurality of couplings, each coupling comprising:
   (i) a connector adapted to receive the fixation element, and
   (ii) a guide adapted to receive the stented valve;
      wherein the connector is reciprocally movable relative to the guide; and
(c) a plurality of respective fixation elements according to the first aspect of the present invention.

The at least one fixation element is a fixation element according to the first aspect of the present invention.

Optionally, the prosthetic valve comprises:
(a) a stented valve;
(b) at least one coupling comprising:
   (i) a connector adapted to receive the fixation element, and
   (ii) a guide adapted to receive the stented valve;
      wherein the connector is reciprocally movable relative to the guide; and
(c) at least one fixation element according to the first aspect of the present invention.

The prosthetic valve comprises: a stented valve, and at least one fixation element according to the first aspect of the present invention, each received by at least one coupling.

The prosthetic valve comprises: a stented valve, and at least one fixation element according to the first aspect of the present invention; wherein the stented valve and at least one fixation element are each received by at least one coupling.

Optionally, the longitudinal axis of the coupling is substantially coaxial with the longitudinal axis of the stented valve. Further optionally, the longitudinal axis of the coupling, the longitudinal axis of the stented valve, and the longitudinal axis of the fixation element are each substantially coaxial.

Optionally, the longitudinal axis of the guide is substantially coaxial with the longitudinal axis of the stented valve. Further optionally, the longitudinal axis of the guide, the longitudinal axis of the stented valve, and the longitudinal axis of the fixation element are each substantially coaxial.

Optionally, the longitudinal axis of the guide, the longitudinal axis of the stented valve, and the longitudinal axis of the control rod of the at least one fixation element, are each substantially coaxial.

Optionally, the at least one coupling and the at least one fixation element are co-localised.

Optionally, the connector of the at least one coupling, and the pivotable clamp of the at least one fixation element, are co-localised.

Optionally, the connector of the at least one coupling is connected to the pivotable clamp of the at least one fixation element. Optionally, the at least one coupling is connected to the fixation element via the connector. Optionally, the at least one coupling is connected to the pivotable clamp via the connector.

Optionally, the connector of the at least one coupling, and the control rod of the at least one fixation element, are co-localised. Optionally, the connector comprises the control rod. Optionally, the control rod comprises the connector.

Optionally, the connector of the at least one coupling is connected to the control rod of the at least one fixation element. Optionally, the at least one coupling is connected to the fixation element via the connector. Optionally, the at least one coupling is connected to the control rod via the connector.

Optionally, the stented valve further comprises at least one commissures post. Optionally, the stented valve further comprises two commissures posts. Optionally, the stented valve further comprises three commissures posts. Optionally, the stented valve further comprises a plurality of commissures posts.

Optionally, the at least one commissures post and the at least one coupling are co-localised.

Optionally, the at least one commissures post and the at least one coupling and the at least one respective fixation element are co-localised.

Optionally, the at least one commissures post is connected to the at least one coupling. Optionally, the at least one commissures post is connected to the at least one coupling via the respective guide.

Optionally, the stented valve comprises an expandable stent scaffold.

Optionally, the guide is adapted to receive the expandable stent scaffold.

Optionally, the guide is adapted to receive the expandable stent scaffold such that the longitudinal axis of the guide is substantially coaxial with the longitudinal axis of the expandable stent scaffold. Further optionally, the longitudinal axis of the guide, the longitudinal axis of the expandable stent scaffold, and the longitudinal axis of the fixation element are each substantially coaxial.

Optionally, the expandable stent scaffold comprises at least one ribbon connected to form a lattice.

Optionally, the expandable stent scaffold comprises resilient material. Optionally, the expandable stent scaffold is formed from resilient material.

Optionally, the at least one ribbon comprises resilient material. Optionally, the at least one ribbon is formed from resilient material.

Optionally, the expandable stent scaffold is formed from a single material. Alternatively, the expandable stent scaffold is formed from several materials.

Optionally, the lattice is manufacturable from a sheet of material, optionally by cutting the sheet of material, optionally by laser cutting the sheet of material.

Optionally, the expandable stent scaffold comprises a metal. Further optionally, the expandable stent scaffold comprises a metal alloy. Further optionally, the expandable stent scaffold comprises a metal alloy of nickel and titanium. Optionally or additionally, the expandable stent scaffold comprises a steel alloy, optionally stainless steel. Further optionally or additionally, the expandable stent scaffold comprises a metal alloy of cobalt and chromium.

Optionally, the expandable stent scaffold is formed from a metal. Further optionally, the expandable stent scaffold is formed from a metal alloy. Further optionally, the expandable stent scaffold is formed from a metal alloy of nickel and titanium. Optionally or additionally, the expandable stent scaffold is formed from a steel alloy, optionally stainless steel. Further optionally or additionally, the expandable stent scaffold is formed from a metal alloy of cobalt and chromium.

Optionally, the expandable stent scaffold comprises a polymer material.

Optionally, the expandable stent scaffold is formed from a polymer material.

Optionally, the expandable stent scaffold comprises polymeric plastic material. Optionally, the expandable stent scaffold comprises organic thermoplastic polymer. Optionally, the expandable stent scaffold comprises polyaryletherketone (PAEK). Optionally, the expandable stent scaffold comprises polyether ether ketone (PEEK).

Optionally or additionally, the expandable stent scaffold comprises synthetic polyester. Further optionally or additionally, the expandable stent scaffold comprises polytetrafluoroethylene (PTFE).

Optionally, the lattice comprises: at least one hinge at a ribbon intersection.

Optionally, the expandable stent scaffold has a substantially tubular shape.

Optionally, the expandable stent scaffold has a funnel shape. Further optionally, the expandable stent scaffold has a funnel shape whereby the diameter of a first open end of the expandable stent scaffold is greater than the diameter of a second open end of the expandable stent scaffold.

Optionally, the expandable stent scaffold has a substantially trumpet-shape.

Optionally, the expandable stent scaffold has a shape that is asymmetrical. Optionally, the expandable stent scaffold has a shape that is axially asymmetrical. Optionally, the first open end of the expandable stent scaffold has a shape that is axially asymmetrical.

Alternatively, the expandable stent scaffold has a shape that is symmetrical. Optionally, the expandable stent scaffold has a shape that is axially symmetrical. Optionally, the first open end of the expandable stent scaffold has a shape that is axially symmetrical.

Optionally, the angle between the expandable stent scaffold and the axis of the expandable stent scaffold varies along the perimeter of the expandable stent scaffold cross-section. Optionally, at least one part of the perimeter of one opening, optionally the first open end, of the expandable stent scaffold may extend further parallel to the axis of the expandable stent scaffold than at least one other part of the perimeter of the same opening, optionally the first open end, of the expandable stent scaffold.

Optionally, the first open end of the expandable stent scaffold defines the stent perimeter.

Optionally, the at least one coupling defines the coupling perimeter, whereby the at least one coupling is contained within the coupling perimeter. Further optionally, the plurality of couplings defines the coupling perimeter, whereby each coupling is contained within the coupling perimeter.

Optionally, the coupling perimeter is shorter than the stent perimeter. Alternatively, the coupling perimeter is longer than the stent perimeter. Alternatively, the coupling perimeter is the same length as the stent perimeter.

Optionally, the at least one fixation element defines the fixation element perimeter, whereby the at least one fixation element is contained within the fixation element perimeter. Further optionally, the plurality of fixation elements defines the fixation element perimeter, whereby each fixation element is contained within the fixation element perimeter.

Optionally, the fixation element perimeter is shorter than the stent perimeter. Alternatively, the fixation element perimeter is longer than the stent perimeter. Alternatively, the fixation element perimeter is the same length as the stent perimeter.

Optionally, the expandable stent scaffold further comprises at least one eyelet connected to the lattice, whereby the at least one eyelet is adjacent to at least one open end of the expandable stent scaffold.

Optionally, the expandable stent scaffold further comprises: a skirt. Optionally, the skirt is inside the expandable stent scaffold frame. Further optionally, the skirt is outside the expandable stent scaffold frame. Further optionally, the skirt is both inside and outside of the expandable stent scaffold frame.

Optionally, the skirt is integrally formed in the expandable stent scaffold.

Optionally, the skirt is attached to the expandable stent scaffold.

Optionally, the skirt is attached to the expandable stent scaffold by an adhesive selected from the group comprising: glue, fixative, gum, cement, bonding, binder, and sealant. Optionally, the skirt is glued to the expandable stent scaffold.

Optionally or additionally, the skirt is attached to the expandable stent scaffold by attachment means selected from the group comprising: stitching, sewing, and suturing.

Optionally or additionally, the skirt is attached to the expandable stent scaffold by attachment means selected from the group comprising welding, heat bonding, brazing, and soldering.

Optionally, the longest dimension of the guide is longer than the longest dimension of the stented valve.

Optionally, the longest dimension of the guide is substantially the same length as the longest dimension of the stented valve.

Optionally, the longest dimension of the guide is shorter than the longest dimension of the stented valve.

Optionally, the longest dimension of the guide is about half the length of the longest dimension of the stented valve.

Optionally or additionally, at least one terminal end of the guide extends beyond a terminal end of the stented valve. Further optionally or additionally, each terminal end of the guide extends beyond each terminal end of the stented valve.

Optionally, the guide extends beyond the stented valve further at one end of the guide than at the other end of the guide.

Alternatively, the stented valve is a valve spacer.

Optionally, the valve spacer is suitable for filling the regurgitant area of a diseased valve.

Optionally, the valve spacer is suitable to act like a valve coaption assist device.

Optionally, the valve spacer comprises a solid volume.

Optionally, the valve spacer is substantially cylindrical. Optionally, the valve spacer is substantially conical. Optionally, the valve spacer is substantially a truncated cone shape. Optionally, the valve spacer is substantially a frustrum shape.

Optionally, the valve spacer is substantially an ellipsoid shape. Optionally, the valve spacer is substantially a prolate spheroid shape.

Optionally, the valve spacer is substantially a kidney bean shape.

Optionally, the valve spacer has an oval cross-section. Optionally, the valve spacer has a kidney bean shaped cross-section.

Optionally, the valve spacer is expandable. Optionally, the valve spacer comprises nitinol. Optionally, the valve spacer comprises nitinol self-expanding mesh. Optionally, the valve spacer is inflatable.

Optionally, the external surface of the valve spacer comprises a soft material.

Optionally, the external surface of the valve spacer comprises a natural material.

Optionally, the external surface of the valve spacer comprises a synthetic material.

Optionally, the external surface of the valve spacer comprises a polymeric material.

Optionally, the external surface of the valve spacer comprises a material selected from the group comprising: plastic, polymeric plastic, polyester, polyethylene terephthalate (PET), polyethylene terephthalate fabric (PET fabric), polytetrafluoroethylene (PTFE), expanded polytetrafluoroethylene (ePTFE), organic thermoplastic polymer, polyaryletherketone (PAEK), and polyether ether ketone (PEEK).

Accordingly disclosed is a prosthetic valve comprising a valve spacer, at least one coupling, and at least one fixation element according to the first aspect of the present invention.

Also disclosed is a method of manufacture of a prosthetic valve according to the third aspect of the present invention.

A method for repairing or replacing a dysfunctional heart valve using a prosthetic valve according to the third aspect of the present invention can comprisethe steps of:
(i) providing a prosthetic valve according to the third aspect of the present invention;
(ii) moving the connector of at least one coupling relative to the guide of the same coupling;
(iii) displacing the pivotable clamp of at least one fixation element between the open position and the closed position by operation of the proximal end of the control rod of the same fixation element; wherein the pivotable clamp captures a native leaflet;
(iv) disconnecting the distal end of the control rod of at least one fixation element from the pivotable clamp of the same fixation element, and withdrawing the control rod
such that the prosthetic valve is anchored in an appropriate position.

A medical device can comprise:
(a) a prosthetic valve according to the third aspect of the present invention, and
(b) a delivery system for transluminally guiding the prosthetic valve and adapted to receive the prosthetic valve.

Optionally, the delivery system comprises a delivery tube adapted to receive the prosthetic valve.

Optionally, the delivery tube comprises a series of retractable telescopic tubes.

Optionally, the medical device further comprises a handle operable to control the expansion and release of the stented valve.

Also disclosed is a method of manufacture of such a medical device.

A method for repairing or replacing a dysfunctional heart valve using such a medical device can comprise the steps of:
(i) providing such a medical device;
(ii) advancing at least part of the delivery system to the native valve;
(iii) unsheathing at least part of the prosthetic valve from the delivery system;
(iv) moving the connector of at least one coupling relative to the guide of the same coupling;
(v) displacing the pivotable clamp of at least one fixation element between the open position and the closed position by operation of the proximal end of the control rod of the same fixation element; wherein the pivotable clamp captures a native leaflet;
(vi) disconnecting the distal end of the control rod of at least one fixation element from the pivotable clamp of the same fixation element, and withdrawing the control rod;
(vii) withdrawing the delivery system;
such that the prosthetic valve is anchored in an appropriate position.

### Brief description of the drawings

Embodiments of the invention will now be described with reference to the accompanying drawings in which:

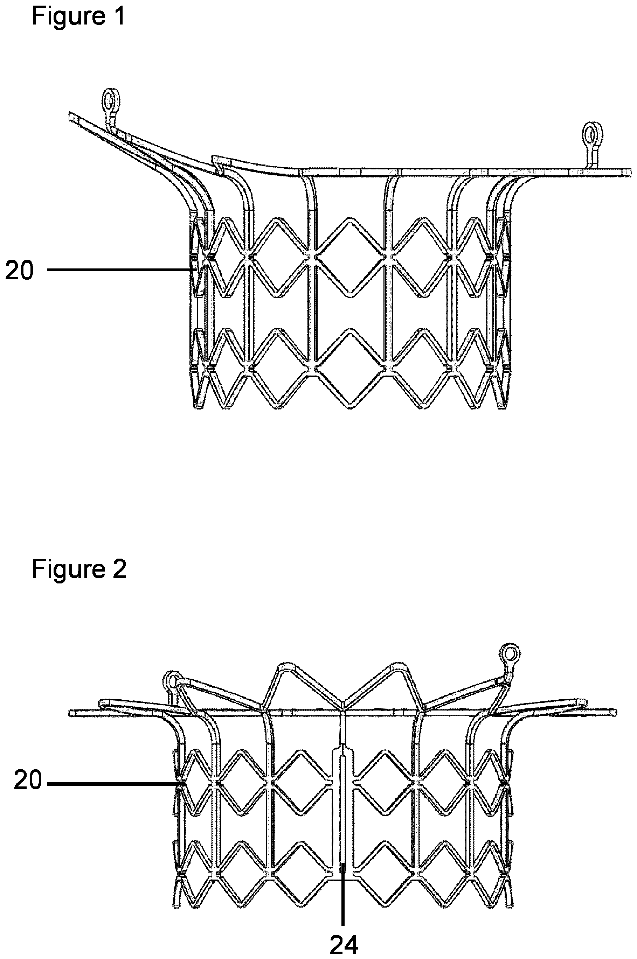

Figure 1 shows a side view of a stented valve comprising an expandable stent scaffold 20 in the expanded configuration;
Figure 2 shows a side view of a stented valve comprising an expandable stent scaffold 20 in the expanded configuration;

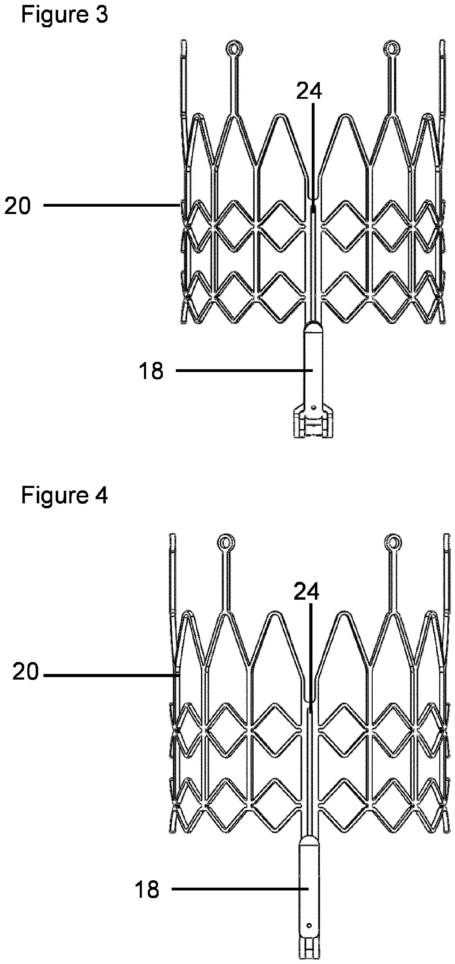

Figure 3 shows a side view of one embodiment of the invention, comprising an expandable stent scaffold 20 in the expanded configuration, and a pivotable clamp 18 comprising an offset hinge; wherein the connector 12 is at the fist end of the slot 24;
Figure 4 shows a side view of one embodiment of the invention, comprising an expandable stent scaffold 20 in the expanded configuration, and a pivotable clamp 18; wherein the connector 12 is at the fist end of the slot 24;

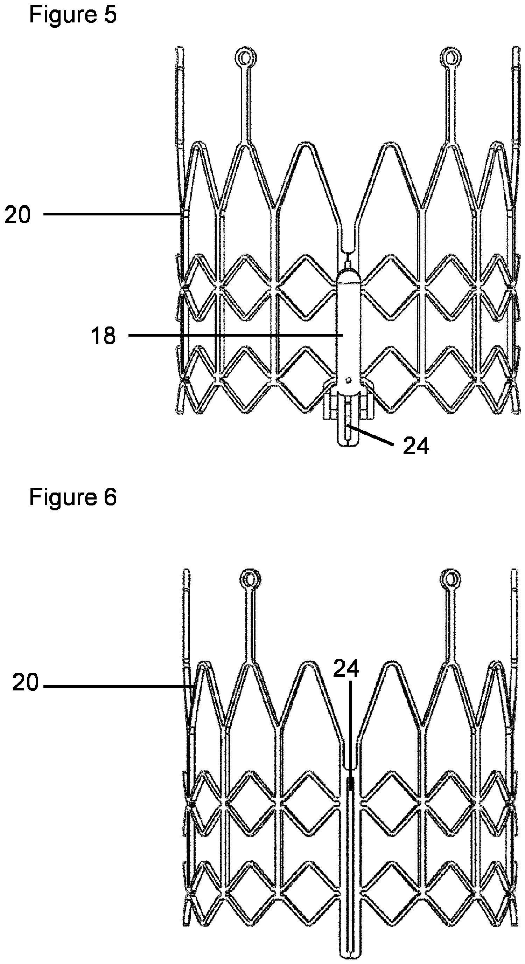

Figure 5 shows a side view of one embodiment of the invention, comprising an expandable stent scaffold 20 in the expanded configuration, and a pivotable clamp 18 comprising an offset hinge; wherein the connector 12 is positioned towards the second end of the slot 24;
Figure 6 shows a side view of one embodiment of the invention, comprising an expandable stent scaffold 20 in the expanded configuration;

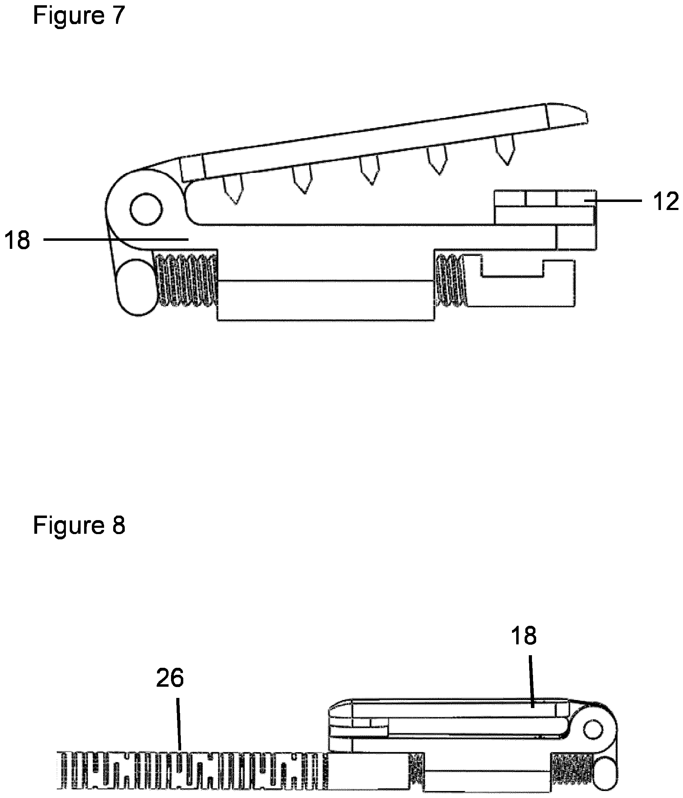

Figure 7 shows a side view of one embodiment of the invention, comprising a pivotable clamp 18;
Figure 8 shows a side view of one embodiment of the invention, comprising a pivotable clamp 18 in the closed position, and the distal end of a control rod; wherein the connection between the distal end of the control rod and the pivotable clamp 18 is covered by the sleeve 26 of the control rod;

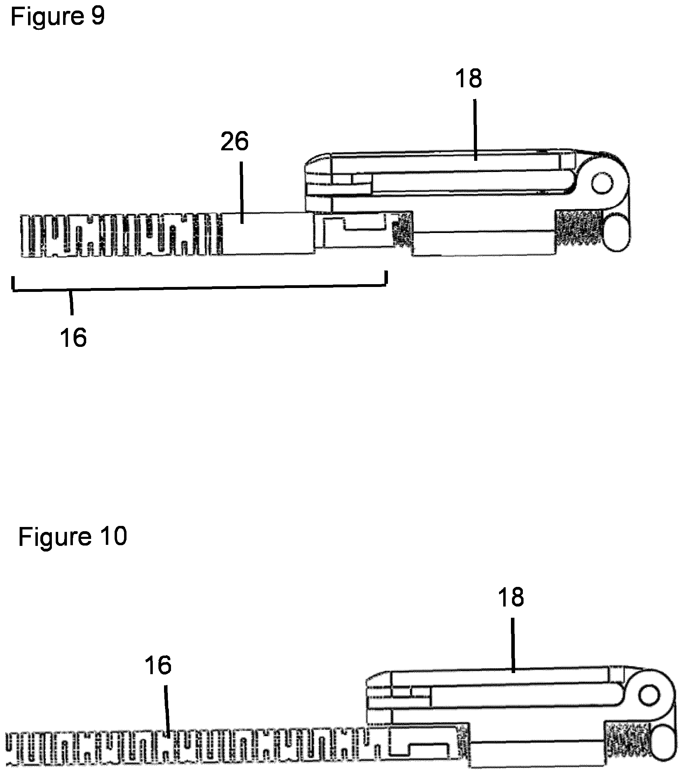

Figure 9 shows a side view of one embodiment of the invention, comprising a pivotable clamp 18 in the closed position, and the distal end of a control rod 16; wherein the connection between the distal end of the control rod 16 and the pivotable clamp 18 is not covered by the sleeve 26 of the control rod 16;
Figure 10 shows a side view of one embodiment of the invention, comprising a pivotable clamp 18 in the closed position, and the distal end of a control rod 16;

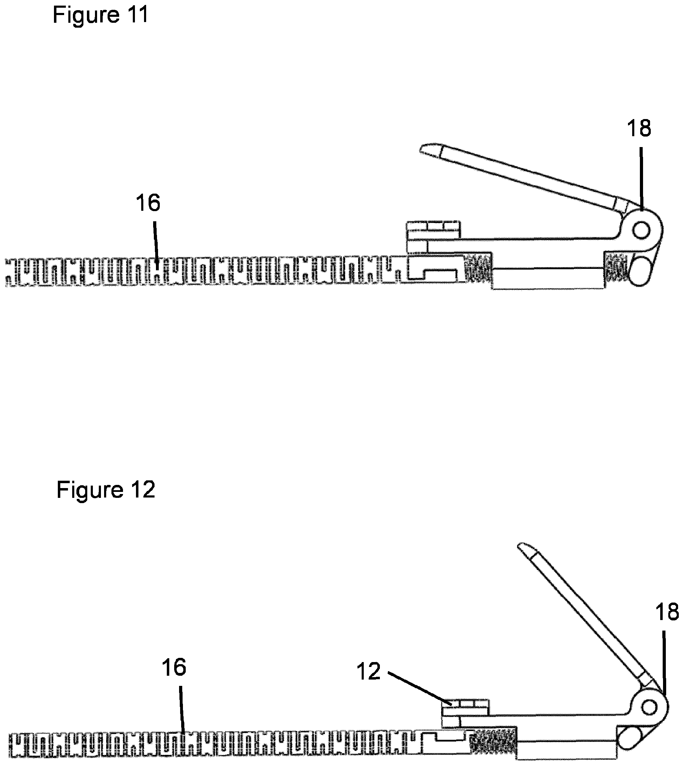

Figure 11 shows a side view of one embodiment of the invention, comprising a pivotable clamp 18, and the distal end of a control rod 16;
Figure 12 shows a side view of one embodiment of the invention, comprising a pivotable clamp 18 in the open position, and the distal end of a control rod 16;

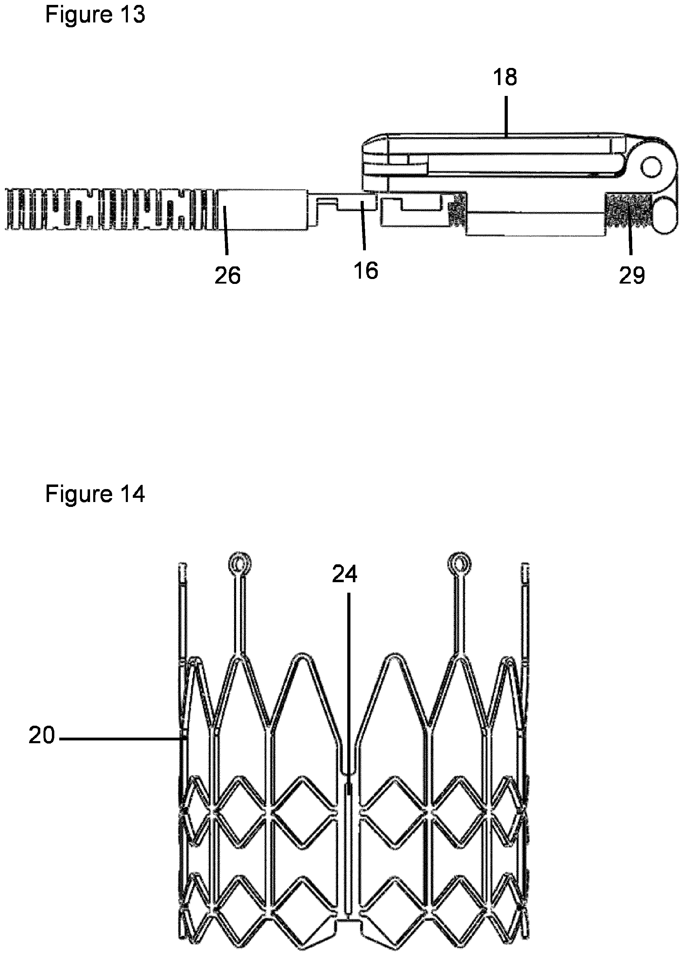

Figure 13 shows a side view of one embodiment of the invention, comprising a pivotable clamp 18 in the closed position, and the distal end of a control rod 16; wherein the distal end of the control rod 16 is not covered by the sleeve 26, wherein the distal end of the control rod 16 and the pivotable clamp 18 are disconnected;
Figure 14 shows a side view of a stented valve comprising an expandable stent scaffold 20 in the expanded configuration;

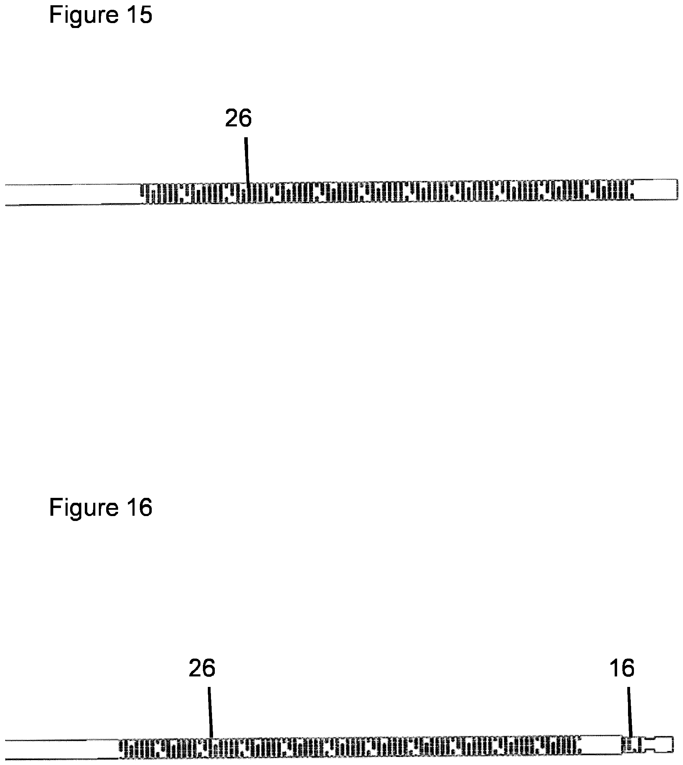

Figure 15 shows a side view of one embodiment of the invention, comprising at least part of a control rod covered by a sleeve 26;
Figure 16 shows a side view of one embodiment of the invention, comprising at least part of a control rod 16, wherein the distal end of the control rod 16 is not covered by the sleeve 26 of the control rod 16;

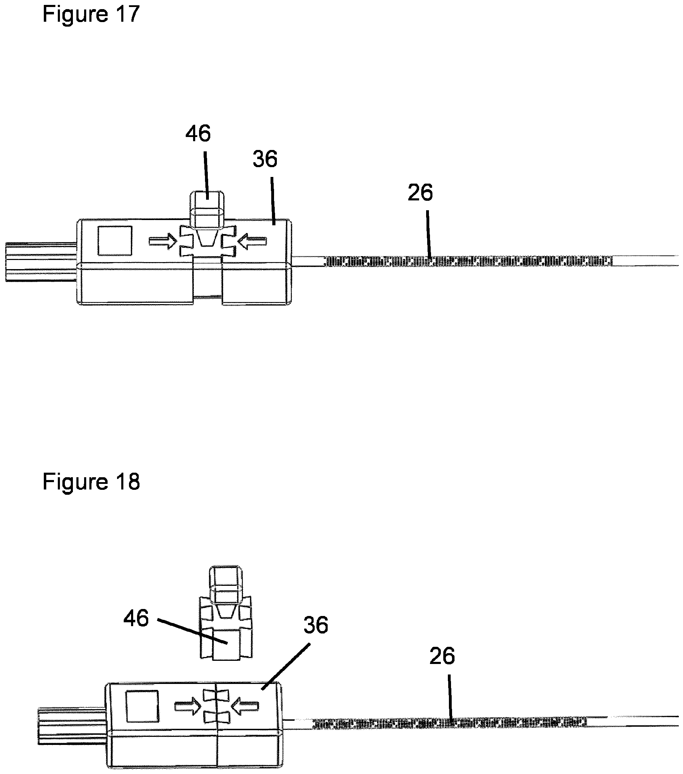

Figure 17 shows a perspective view of one embodiment of the invention, comprising at least part of a control rod 16, and a controller 36;
Figure 18 shows a perspective view of one embodiment of the invention, comprising at least part of a control rod 16, and a controller 36;

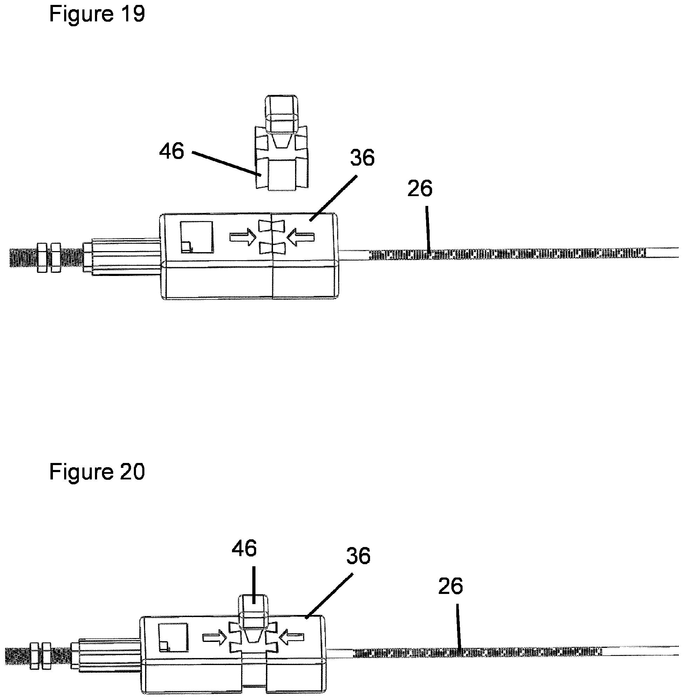

Figure 19 shows a perspective view of one embodiment of the invention, comprising at least part of a control rod 16, and a controller 36;
Figure 20 shows a perspective view of one embodiment of the invention, comprising at least part of a control rod 16, and a controller 36;

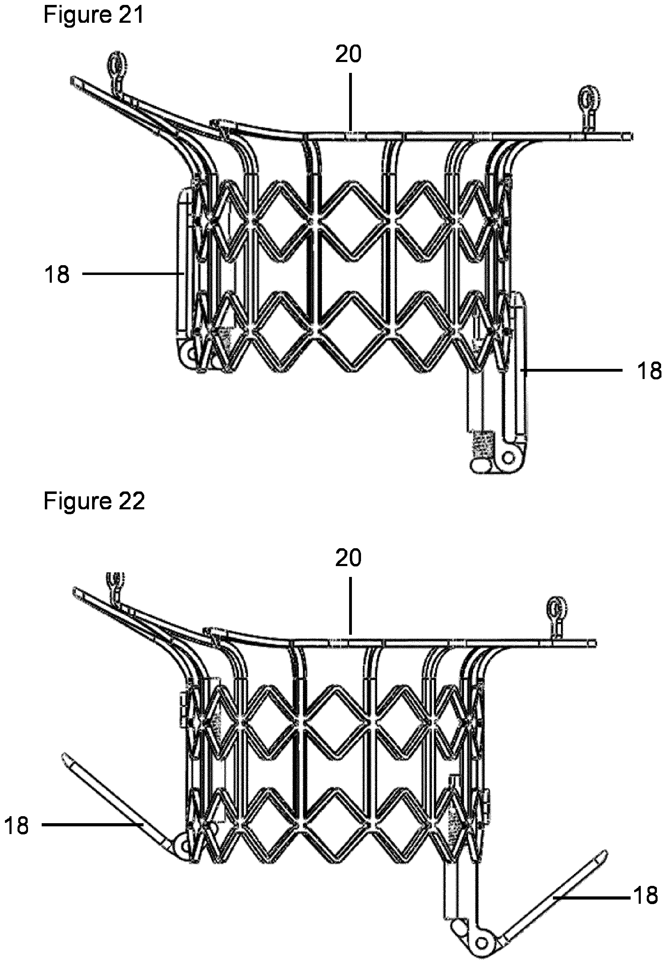

Figure 21 shows a side view of one embodiment of the invention, comprising an expandable stent scaffold 20 in the expanded configuration, and two pivotable clamps 18 in the closed position, wherein each pivotable clamp 18 has a respective coupling, and wherein one connector is at the first end of the respective slot and the other coupling is at the second end of the respective slot;
Figure 22 shows a side view of one embodiment of the invention, comprising an expandable stent scaffold 20 in the expanded configuration, and two pivotable clamps 18 in the open position, wherein each pivotable clamp 18 has a respective coupling, and wherein one connector is at the first end of the respective slot and the other connector is at the second end of the respective slot;

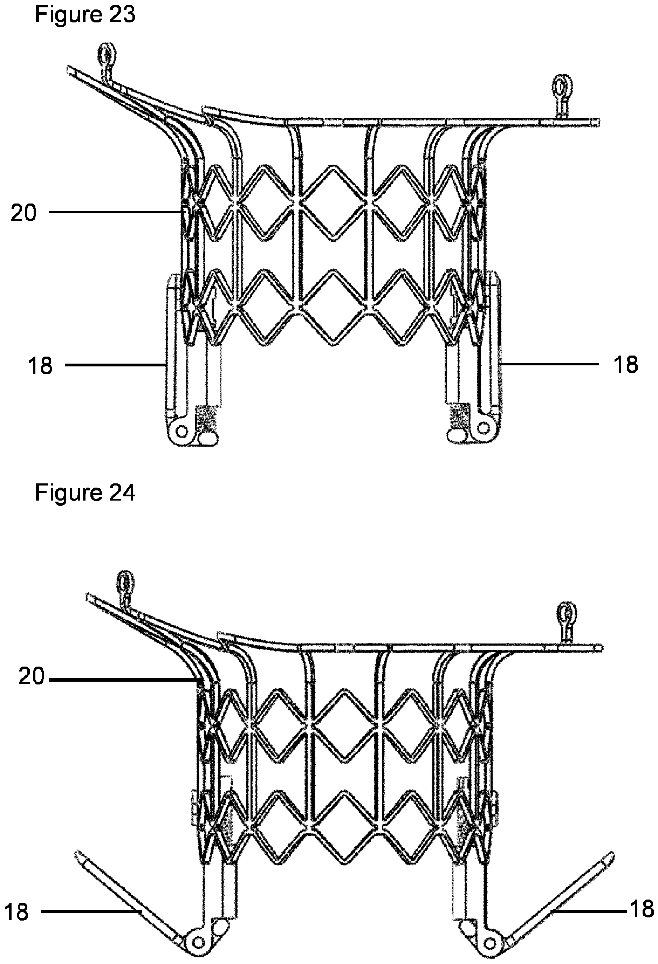

Figure 23 shows a side view of one embodiment of the invention, comprising an expandable stent scaffold 20 in the expanded configuration, and two pivotable clamps 18 in the closed position, wherein each pivotable clamp 18 has a respective coupling, and wherein each connector is at the first end of the respective slot;
Figure 24 shows a side view of one embodiment of the invention, comprising an expandable stent scaffold 20 in the expanded configuration, and two pivotable clamps 18 in the open position, wherein each pivotable clamp 18 has a respective coupling, and wherein each connector is at the first end of the respective slot;

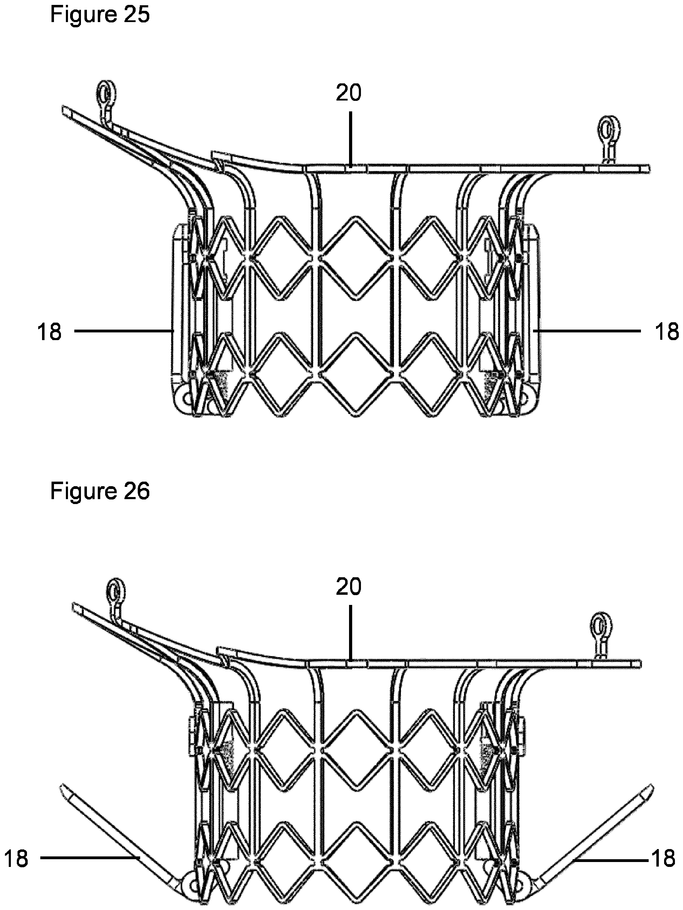

Figure 25 shows a side view of one embodiment of the invention, comprising an expandable stent scaffold 20 in the expanded configuration, and two pivotable clamps 18 in the closed position, wherein each pivotable clamp 18 has a respective coupling, and wherein each connector is at the second end of the respective slot;
Figure 26 shows a side view of one embodiment of the invention, comprising an expandable stent scaffold 20 in the expanded configuration, and two pivotable clamps 18 in the open position, wherein each pivotable clamp 18 has a respective coupling, and wherein each connector is at the second end of the respective slot;

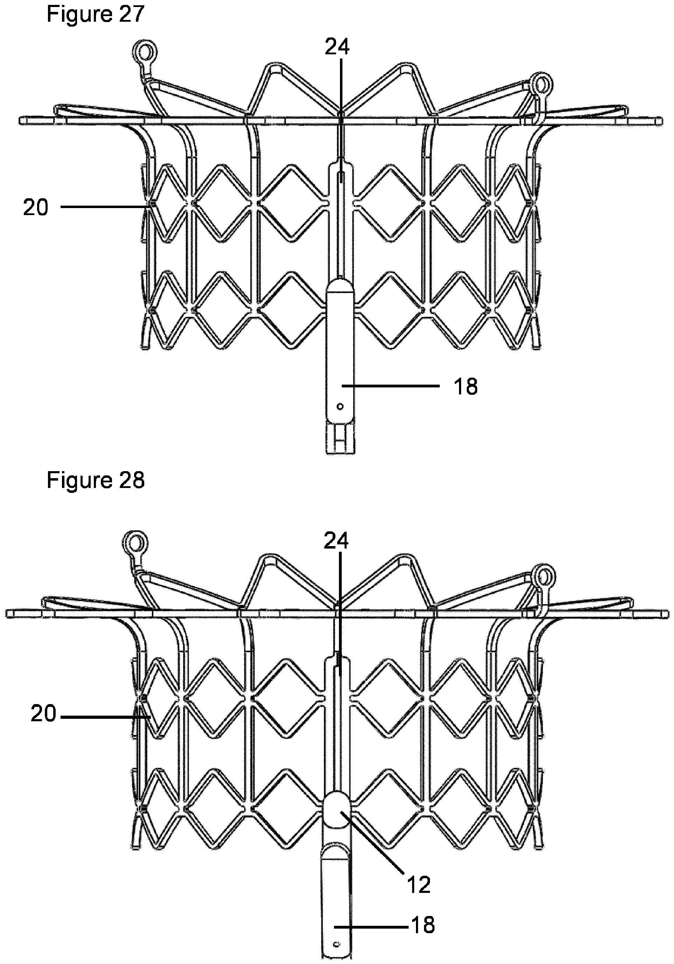

Figure 27 shows a side view of one embodiment of the invention, comprising an expandable stent scaffold 20 in the expanded configuration, and a pivotable clamp 18 in the closed position, wherein the connector is at the first end of the slot 24;
Figure 28 shows a side view of one embodiment of the invention, comprising an expandable stent scaffold 20 in the expanded configuration, and a pivotable clamp 18 in the open position, and the connector 12 at the first end of the slot 24;

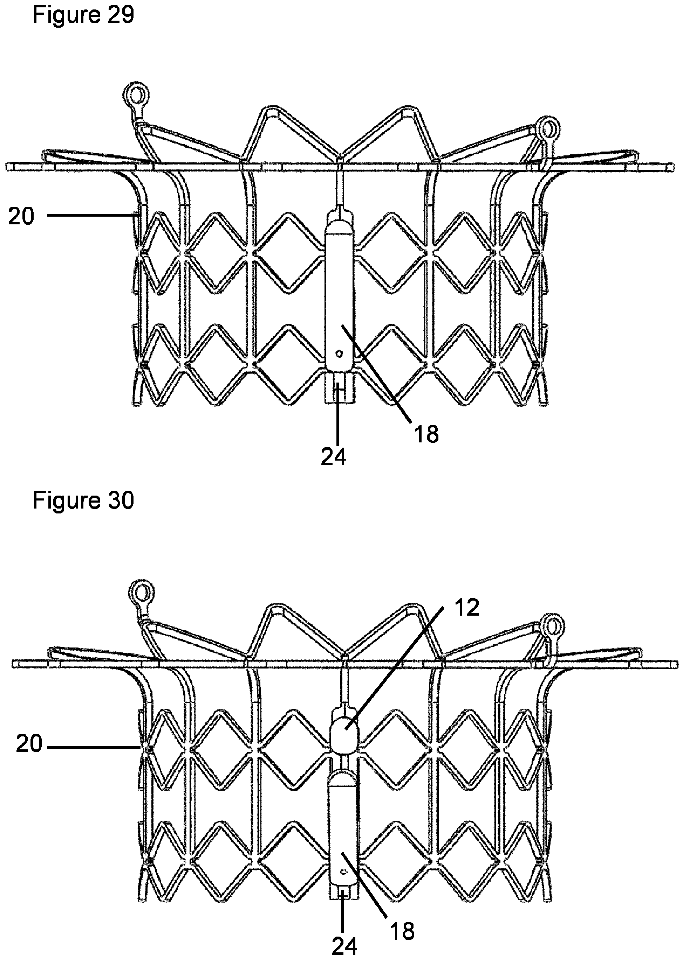

Figure 29 shows a side view of one embodiment of the invention, comprising an expandable stent scaffold 20 in the expanded configuration, and a pivotable clamp 18 in the closed position, wherein the connector is at the second end of the slot 24;
Figure 30 shows a side view of one embodiment of the invention, comprising an expandable stent scaffold 20 in the expanded configuration, and a pivotable clamp 18 in the open position, and the connector 12 at the second end of the slot 24;

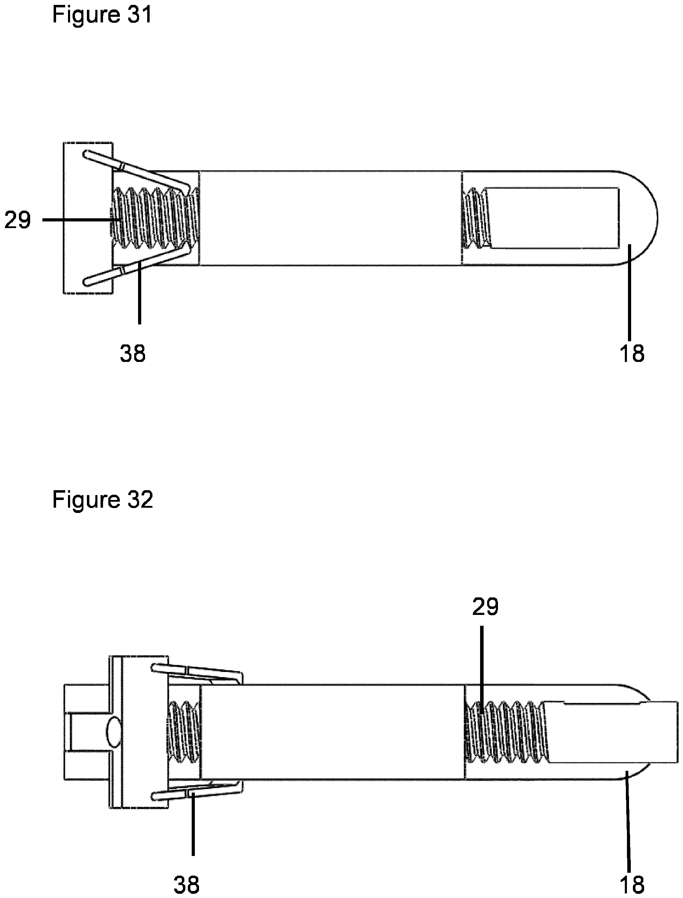

Figure 31 shows a side view of one embodiment of the invention, comprising a pivotable clamp 18 in the locked configuration;
Figure 32 shows a side view of one embodiment of the invention, comprising a pivotable clamp 18 not in the locked configuration;

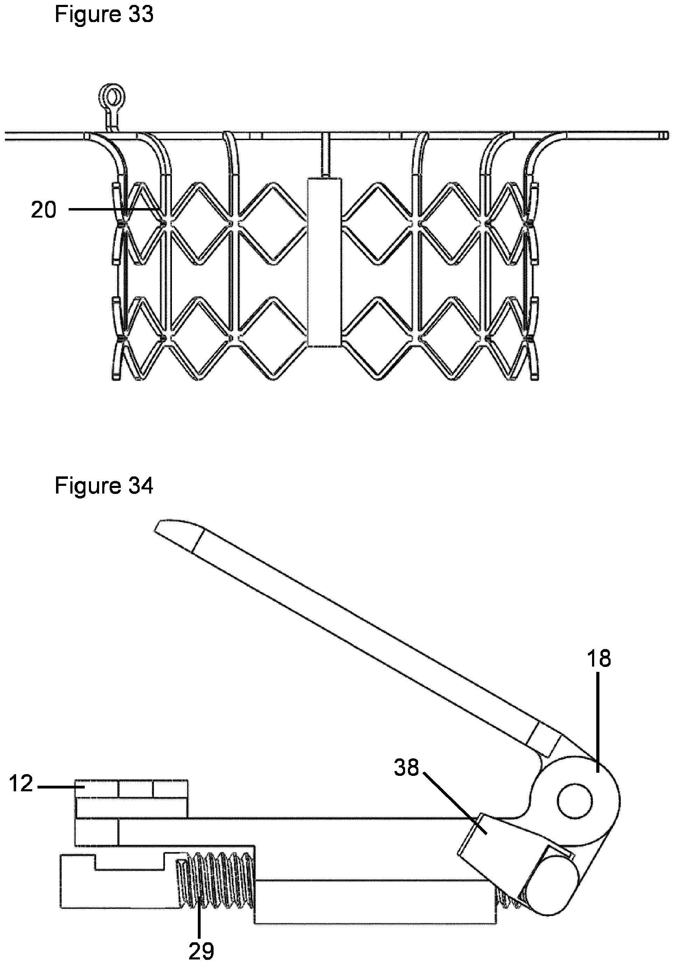

Figure 33 shows a cross section of one embodiment of the invention, comprising an expandable stent scaffold 20 in the expanded configuration;
Figure 34 shows a side view of one embodiment of the invention, comprising a pivotable clamp 18 not in the locked configuration;

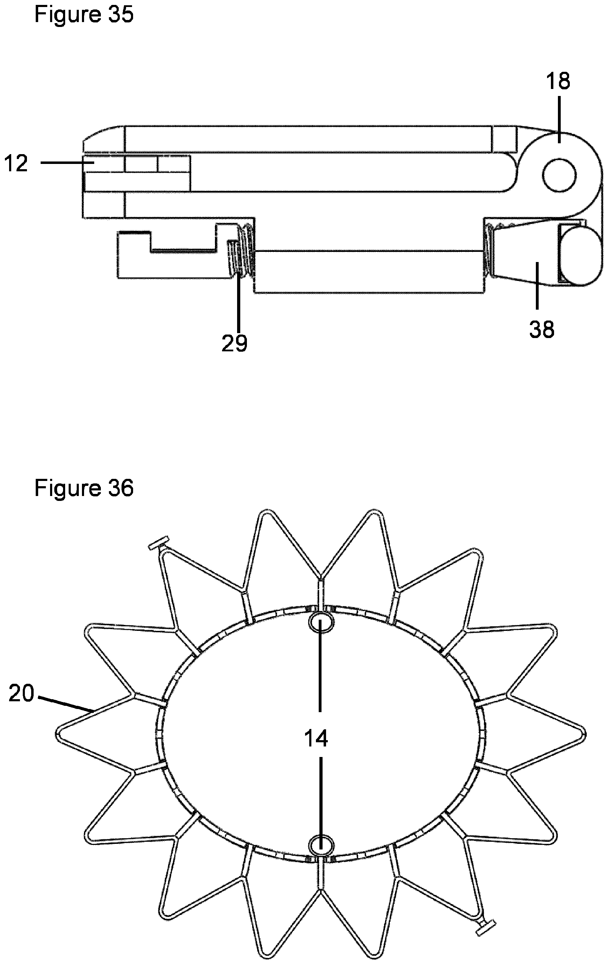

Figure 35 shows a side view of one embodiment of the invention, comprising a pivotable clamp 18 in the locked configuration;
Figure 36 shows a top view of one embodiment of the invention, comprising an expandable stent scaffold 20 in the expanded configuration, and two guides 14 each comprising a tube, wherein each tube has an oval cross section;

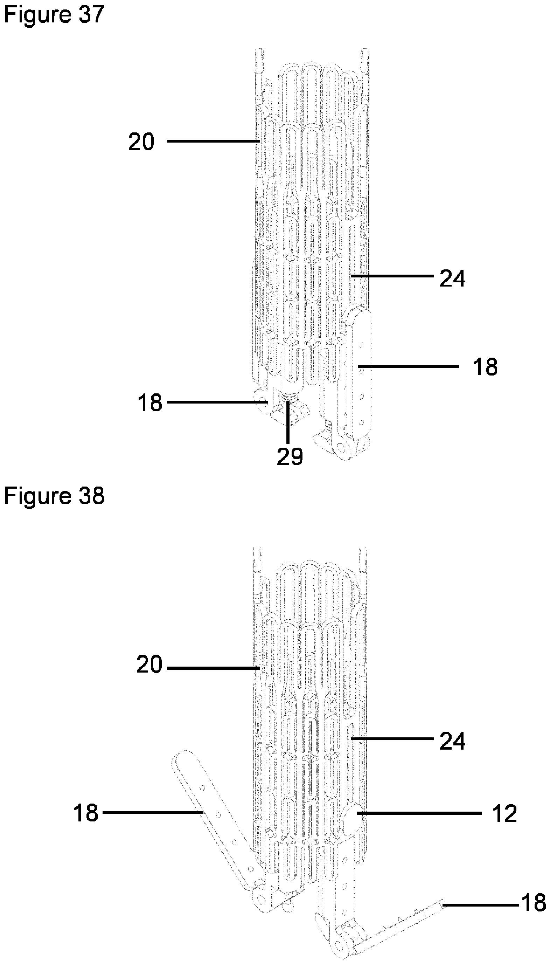

Figure 37 shows a perspective view of one embodiment of the invention, comprising a contracted expandable stent scaffold 20, two pivotable clamps 18 in the closed position, wherein each pivotable clamp 18 has a respective coupling, and wherein each connector is at the first end of the slot 24;
Figure 38 shows a perspective view of one embodiment of the invention, comprising a contracted expandable stent scaffold 20, two pivotable clamps 18 in the open position, wherein each pivotable clamp 18 has a respective coupling, and wherein each connector 12 is at the first end of the slot 24;

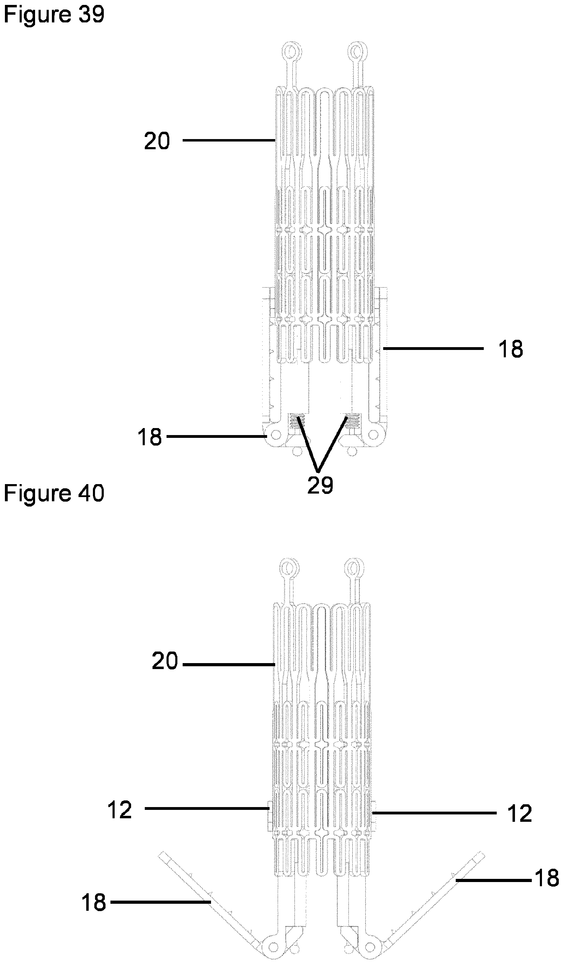

Figure 39 shows a side view of one embodiment of the invention, comprising a contracted expandable stent scaffold 20, two pivotable clamps 18 in the closed position, wherein each pivotable clamp 18 has a respective coupling, and wherein each connector is at the first end of the slot 24;
Figure 40 shows a side view of one embodiment of the invention, comprising a contracted expandable stent scaffold 20, two pivotable clamps 18 in the open position, wherein each pivotable clamp 18 has a respective coupling, and wherein each connector 12 is at the first end of the slot 24;

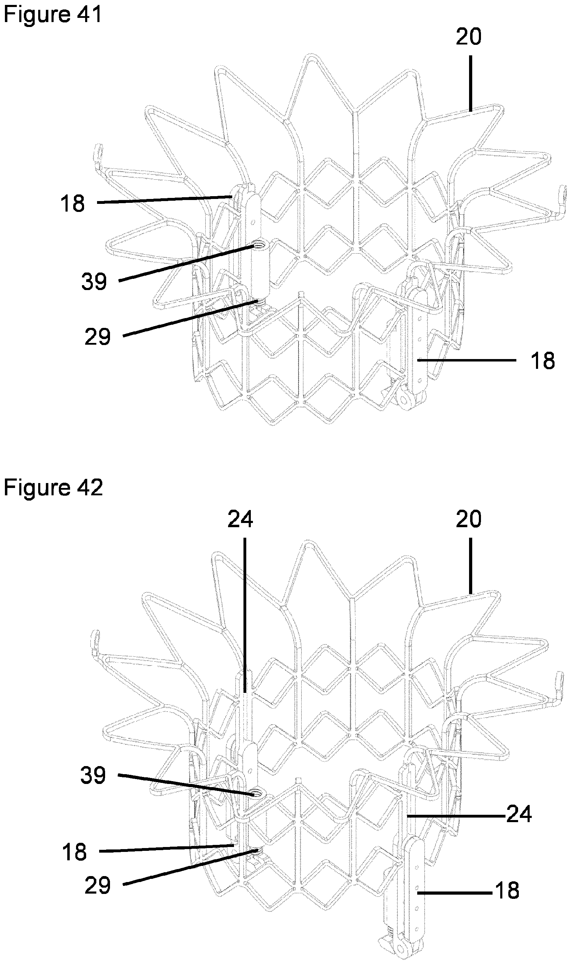

Figure 41 shows a perspective view of one embodiment of the invention, comprising an expandable stent scaffold 20 in the expanded configuration, two pivotable clamps 18 in the closed position, wherein each pivotable clamp 18 has a respective coupling, and wherein each connector is at the second end of the slot;
Figure 42 shows a perspective view of one embodiment of the invention, comprising an expandable stent scaffold 20 in the expanded configuration, two pivotable clamps 18 in the closed position, wherein each pivotable clamp 18 has a respective coupling, and wherein each connector is at the first end of the slot 24;

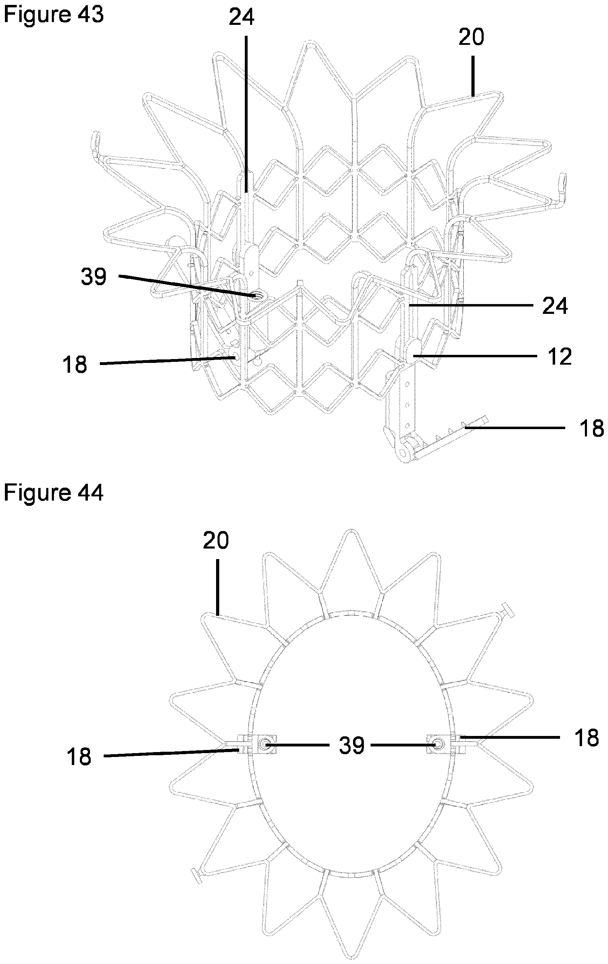

Figure 43 shows a perspective view of one embodiment of the invention, comprising an expandable stent scaffold 20 in the expanded configuration, two pivotable clamps 18 in the open position, wherein each pivotable clamp 18 has a respective coupling, and wherein each connector 12 is at the first end of the slot 24;
Figure 44 shows a top view of one embodiment of the invention, comprising an expandable stent scaffold 20 in the expanded configuration, and two pivotable clamps 18 in the closed position;

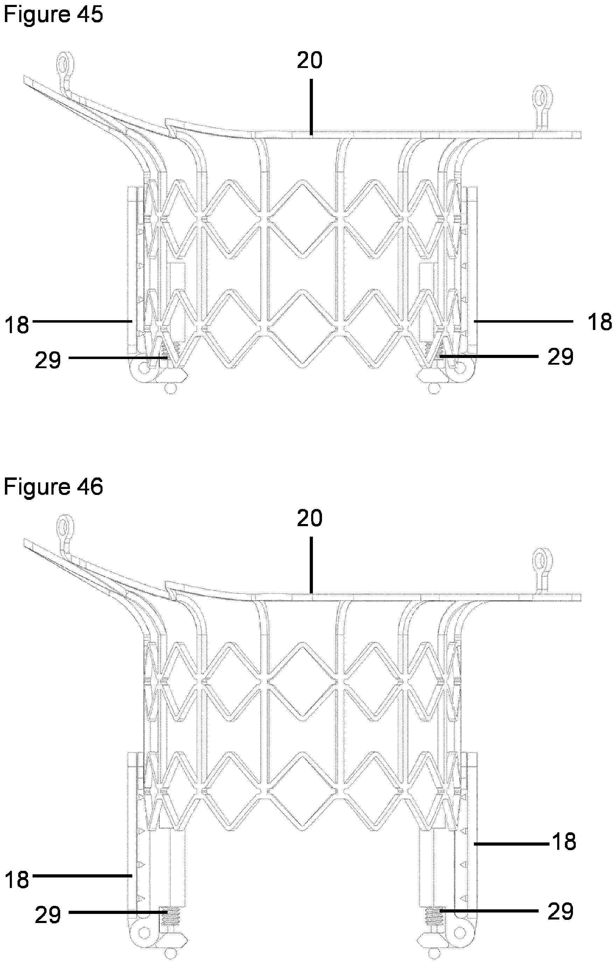

Figure 45 shows a side view of one embodiment of the invention, comprising an expandable stent scaffold 20 in the expanded configuration, two pivotable clamps 18 in the closed position, wherein each pivotable clamp 18 has a respective coupling, and wherein each connector is at the second end of the slot 24;
Figure 46 shows a side view of one embodiment of the invention, comprising an expandable stent scaffold 20 in the expanded configuration, two pivotable clamps 18 in the closed position, wherein each pivotable clamp 18 has a respective coupling, and wherein each connector is at the first end of the slot;

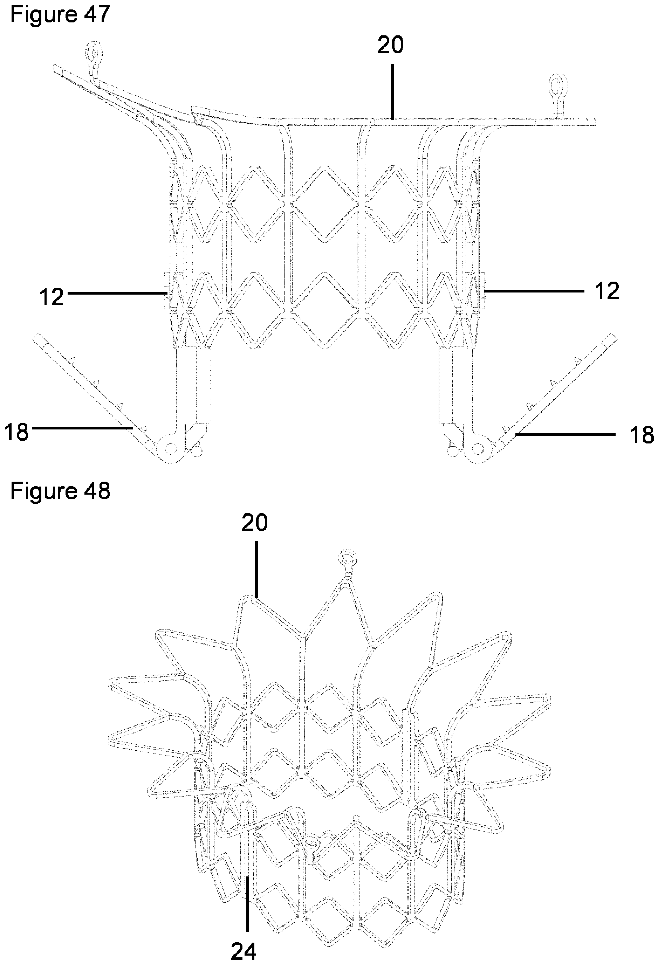

Figure 47 shows a side view of one embodiment of the invention, comprising an expandable stent scaffold 20 in the expanded configuration, two pivotable clamps 18 in the open position, wherein each pivotable clamp 18 has a respective coupling, and wherein each connector 12 is at the first end of the slot;
Figure 48 shows a perspective view of a stented valve comprising an expandable stent scaffold 20 in the expanded configuration;

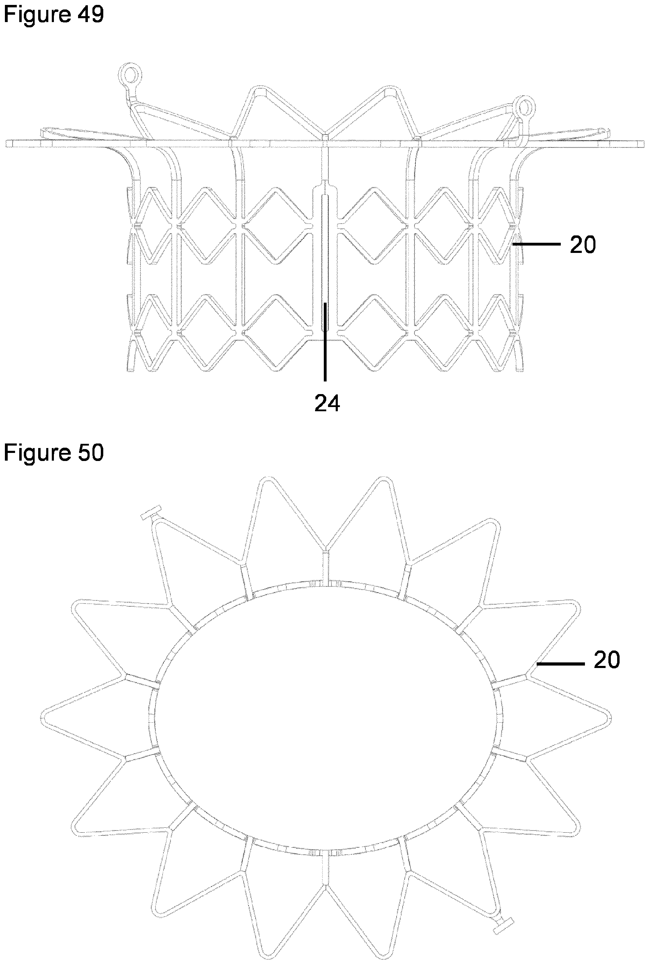

Figure 49 shows a side view of a stented valve comprising an expandable stent scaffold 20 in the expanded configuration;
Figure 50 shows a top view of a stented valve comprising an expandable stent scaffold 20 in the expanded configuration;

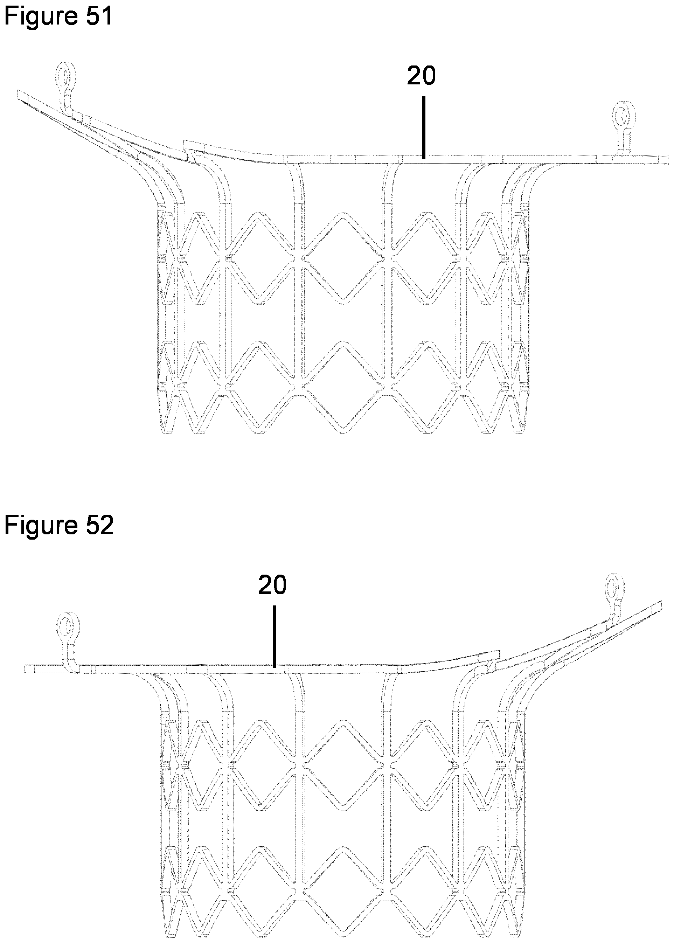

Figure 51 shows a side view of a stented valve comprising an expandable stent scaffold 20 in the expanded configuration;
Figure 52 shows a side view of a stented valve comprising an expandable stent scaffold 20 in the expanded configuration;

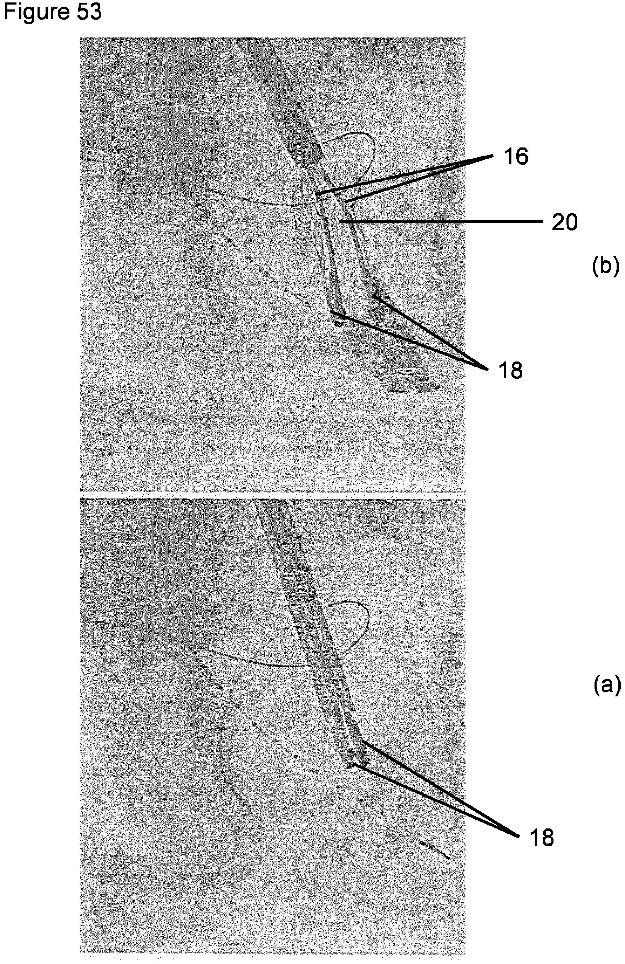

Figure 53 shows x-ray images of an embodiment of the invention in use in an animal model, in (a) the pivotable clamps 18 have been unsheathed from the delivery tube, in (b) the pivotable clamps 18 and part of the expandable stent scaffold 20 have been unsheathed an expandable stent scaffold 20 in the expanded configuration;

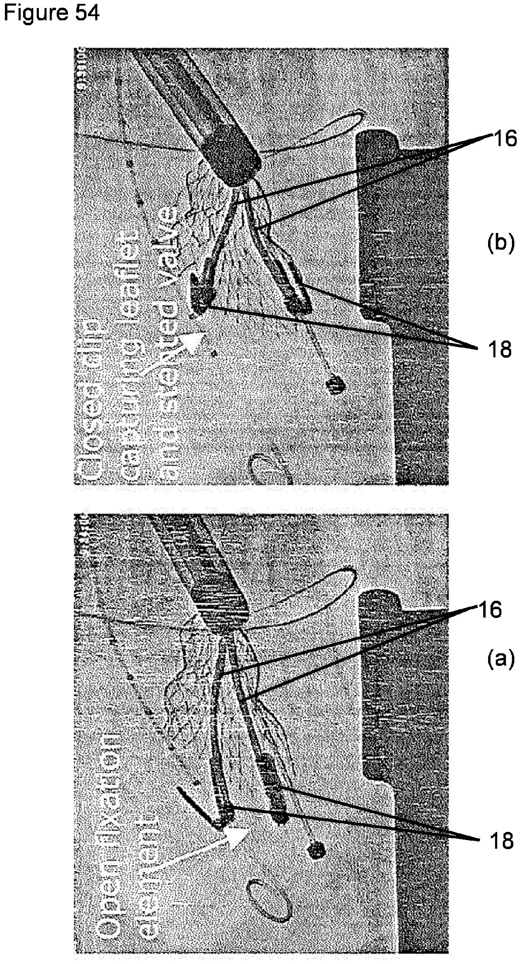

Figure 54 shows x-ray images of an embodiment of the invention in landscape orientation, in (a) the pivotable clamp 18 close to the white arrow and associated white text "Open fixation element", is in the open position, in (b) the pivotable clamp 18 close to the white arrow and associated white text "Closed clip capturing leaflet and stented valve", is in the closed position, capturing the native leaflet;

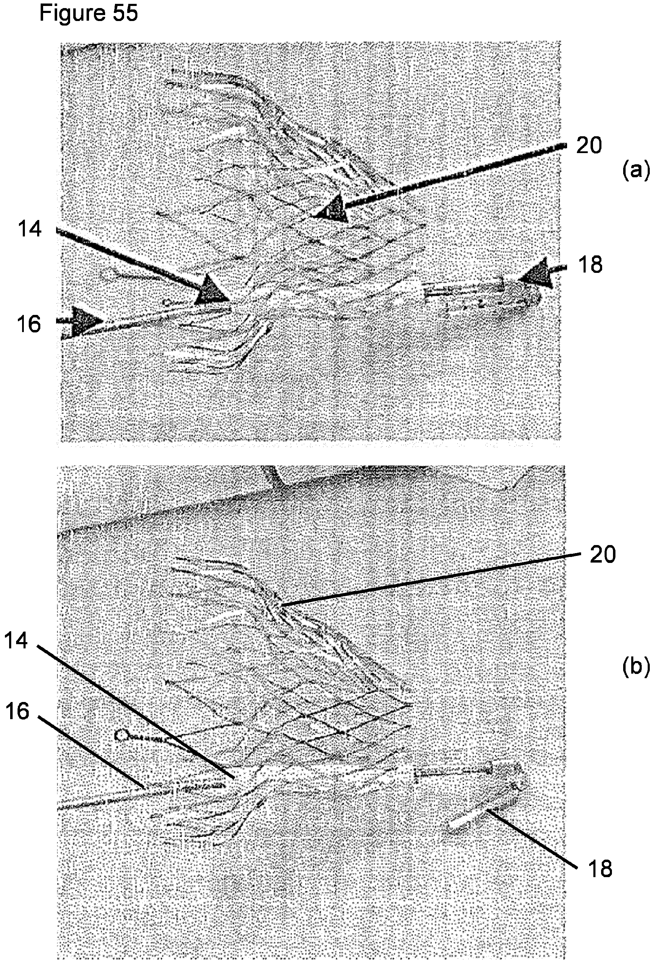

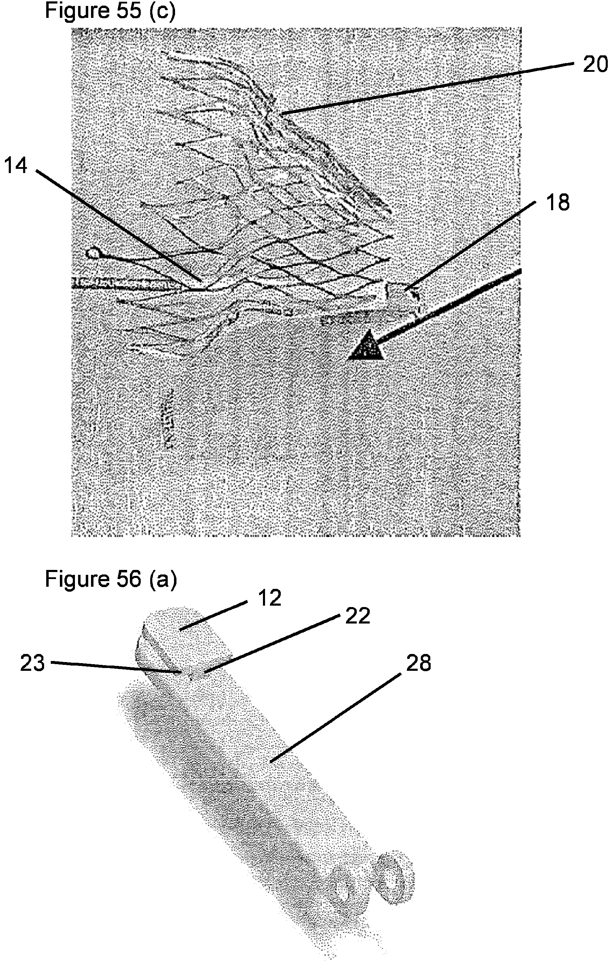

Figure 55 shows images of an embodiment of the invention outside of an animal model, (a) shows a expandable stent scaffold 20 connected to a guide 14 comprising a tube, wherein a control rod 16 comprises the connector, and passes through the tube lumen and connects to a pivotable clamp 18 in the closed position, (b) shows the pivotable clamp 18 in the open position wherein the connector is at the first end of the guide 14, (c) shows the pivotable clamp 18 in the closed position wherein the connector is at the second end of the guide 14, a simulated native leaflet (piece of paper) is indicated by the black arrow, and the piece of paper and the expandable stent scaffold 20 are captured between the arms of the pivotable clamp 18;

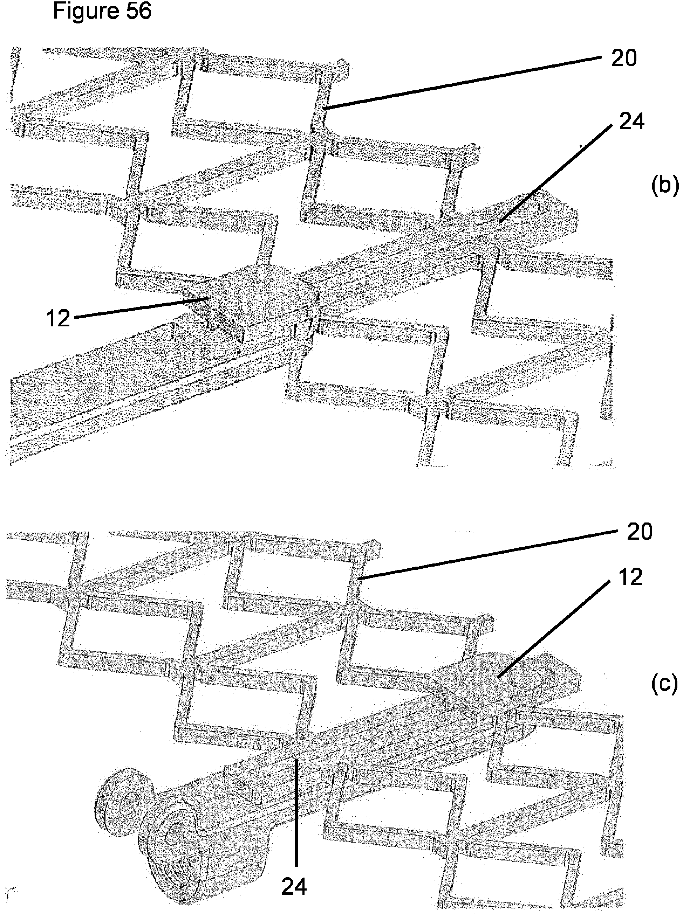

Figure 56 shows perspective views of one embodiment of the invention, (a) shows one arm 28 of a pivotable clamp and a connector 12, (b) shows a coupling wherein the guide is a slot 24 in the expandable stent scaffold 20 and the connector 12 comprises a projection 22 and a stop 23, (c) shows a coupling wherein the guide 14 is a slot 24 in the expandable stent scaffold 20 and the connector 12 comprises a projection 22 and a stop 23, wherein the connector 12 is connected to one arm 28 of a pivotable clamp 18;

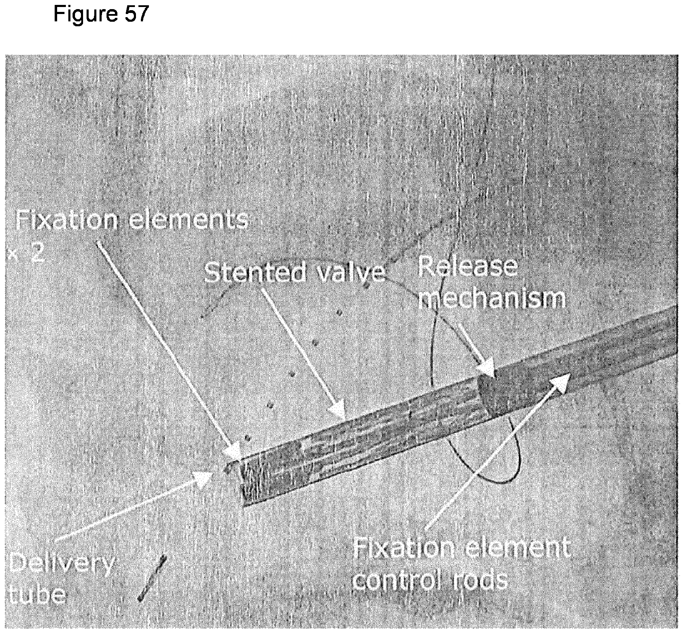

Figure 57 shows an x-ray image of one embodiment of the invention in a sheep model, in this figure several parts of the embodiments shown are indicated by white arrows and associated white text: the white text "Fixation elements x 2" refers to one or more pivotable clamps 18, the white text "Stented valve" refers to a stented valve, the white text "Fixation element control rods" refers to control rods 16, the white text "Delivery tube" refers to a delivery tube, the white text "Release mechanism" refers to part of the delivery system.

### Examples

### Example 1 - pre-clinical use in an animal model

1. Perform sternotomy, expose the heart and locate ideal access site in the left atrium for crossing of the prosthetic valve with the delivery system. This approach mimics a superior trans-septal clinical situation
2. Place purse-string sutures in the left atrium at the site of access
3. Under angiogram guidance, place a wire in the coronary sinus
4. Place pig-tail catheter in the apex of the left ventricle
5. Perform a left ventricleogram
6. Align the X-ray equipment to achieve an A2-P2 long axis view through the left side of the heart
7. Place transesophageal echocardiography probe in the correct position to achieve a long axis view of the left atrium and left ventricle with the plane of the probe lined up through the A2 and P2 hinge points of the valve, or use an intra cardiac echo probe
8. Using transesophageal echocardiography estimate the length of the A2 section of the mitral valve from tip to hinge point, estimate the length of the P2 section of the mitral valve from tip to hinge point
9. Identify the annulus plane for delivery of the plane of the implant to line up with, this should be confirmed on both angiogram and transesophageal echocardiography. Can pull back pig-tail catheter and place in the non-coronary cusp of the aortic root at this point to give additional marker for valve location
10. Perform a final confirmation of the annulus plane on both transesophageal echocardiography and angiogram
11. Gain access to the left atrium and place wire across the mitrial valve
12. Advance the delivery system into the left atrium and into the left ventricle
13. Confirm that the delivery system is free from chordal entanglement
14. Set rotation of the delivery system to align the anterior pivotable clamp with the anterior leaflet and the posterior pivotable clamp with the P2 segment of the posterior valve. Use pivotable clamp opening and closing mechanism for this alignment.
15. Determine the height of the delivery system to ensure that the stented valve aligns with the hinge point of the native leaflet. Advance forward 5 mm from this point.
16. Double check height and rotation
17. Start to unsheathe the stented valve and the delivery system
18. Unsheathe to expose the two clips
19. Check rotation and height once more
20. If happy proceed with unsheathing
21. Continue to unsheathe until the ventricular section is unsheathed
22. Check height once more
23. Continue to unsheathe and stop prior to eyelet release
24. Unscrew anterior leaflet pivotable clamp to open it up
25. Pull back anterior clip
26. Secure anterior pivotable clamp by displacing into the locked configuration and view closely on transesophageal echocardiography and angiogram to check if leaflet capture has been achieved
27. Check under transesophageal echocardiography if anterior leaflet captured
28. If good continue if not unscrew and try and recapture by release and push forward of clip
29. Keep performing steps 27 -31 until happy leaflet capture has been achieved
30. Repeat steps 27-32 for posterior leaflet capture components
31. Pull spacer out of posterior leaflet fixation system
32. Pull back 2.0mm tube grip
33. Release fixation system for posterior leaflet from the leaflet and remove
34. Repeat steps 34-36 for anterior leaflet fixation system
35. Remove delivery system
36. Pull purse-string sutures to close atrium

### Detailed description of the invention

The inventors have developed a medical device for minimally invasive replacement of a dysfunctional mitral valve (or tricuspid valve). In some embodiments, the medical device has been designed as a concise unit that allows deployment and fixation of a stented valve to occur by manipulating the controls at the proximal end of at least one control rod 16. In some embodiments, the medical device has been designed to be delivered from a superior approach, either transeptally or transatrially. However, modifications to the design could switch the delivery route to transapical.

The invention can be used for replacement of a patient's dysfunctional mitral valve in a minimally invasive manner by expanding a stented valve, for example an expandable stent scaffold 20, at the site of the patient's dysfunctional native mitral valve annulus. This stented valve is subsequently attached to the native valve via a number of fixation elements, comprising pivotable clamps 18 that can be independently controlled using a control rod 16, the distal end of the control rod 16 being reversibly connected to the fixation element, and the proximal end of the control rod 16 being located outside of the patient's body. The delivery and fixation of the valve are conducted under x-ray and/or ultrasound guidance.

In at least one embodiment, the invention has some or all of the following components:
A stented valve, comprising: a metallic or polymer expandable stent scaffold 20, and leaflet material, and in some embodiments a skirt material is also incorporated. The expandable stent scaffold 20 can be made of a lattice having a substantially tubular shape or oval or elliptical and defining a lumen. In some embodiments the expandable stent scaffold 20 has a funnel shape, or a trumpet-shape, at one end. In some embodiments there is at least one eyelet adjacent to one opening of the expandable stent scaffold 20.

In some embodiments, one opening of the expandable stent scaffold 20 can have an axially asymmetrical shape, for example such that the shape is a better fit to the native valve. In some embodiments, the slope or angle of the expandable stent scaffold 20 relative to its axis varies along the perimeter of the expandable stent scaffold 20 cross-section. In some embodiments, at least one part of the perimeter of one opening of the expandable stent scaffold 20 may extend further parallel to the axis of the expandable stent scaffold 20 than at least one other part of the perimeter of the same opening of the expandable stent scaffold 20.

In some embodiments, the expandable stent scaffold 20 is formed from a single material. Alternatively, in other embodiments, the expandable stent scaffold 20 is formed from several materials.

In some embodiments the lattice may be manufactured from a sheet of material, for example by cutting the sheet of material, optionally by laser cutting the sheet of material.

There is at least one fixation element. Each fixation element comprises a pivotable clamp 18. In some embodiments, when the fixation element is in operation one side of the clamp "v" pulls on the ventricular side of the native leaflet with its inner surface, while the other side of the "v" engages the inside of the stented valve. The "v' closes via a pivot mechanism and features that can aid closure of the fixation element are located near the pivot. When the fixation element is in the closed position the stented valve and the native leaflet are clamped together.

At least one control rod 16 is reversibly connected to the pivotable clamp 18, such that the control rod 16 controls the clamp.

A medical device comprising: a delivery tube or catheter having an inner lumen suitable for the stented valve to pass through in a contracted state, comprising a series of retractable telescopic tubes, a handle operable to control the expansion and release of the stented valve. Furthermore mechanisms within the handle, or a separate controller 36, can independently advance, retract, open and close the fixation elements, by interacting with the proximal end of the at least one control rod 16.

The key to the process is that there is at least one guide 14 that receives the stented valve or expandable stent scaffold 20, for example the guide 14 may be inbuilt on the stented valve or expandable stent scaffold 20. A connector 12 is reciprocally movable relative to the guide 14, for example the connector 12 may be in communication with the guide 14. The connector 12 receives the fixation element, for example: pivotable clamp 18. The connector 12 and the guide 14 allow the stented valve and the fixation element(s) to be coupled together. The coupling, comprising the guide 14 and the connector 12, does not fix the fixation element in one specific location, however the coupling does allow the fixation element, for example: pivotable clamp 18, to move along a specific path relative to the stented valve or expandable stent scaffold 20, furthermore the coupling ensures that the fixation element will always be located in a position known to the operator/cardiologist. In at least one embodiment, the invention may comprise at least one coupling for a stented valve and the fixation element according to a first aspect of the present invention, the coupling comprising: a connector adapted to receive the fixation element, and a guide adapted to receive the stented valve: wherein the connector is reciprocally movable relative to the guide.

The invention comprises a fixation element comprising the control rod 16 reversibly connected to the pivotable clamp 18. The fixation element may also be referred to as a fastener, and likewise the term fastener may refer to a fixation element. An attachment comprises the fixation element received by the coupling. In at least one embodiment, the control rod 16 and pivotable clamp 18 are configured to facilitate opening and closing of the pivotable clamp 18 irrespective of the state of expansion of the stented valve or expandable stent scaffold 20.

In some embodiments the fixation elements, comprising for example: pivotable clamps 18 may comprise in addition at least one resilient arm, for example at least one securing means, which in at least one embodiment is made of nickel titanium [nitinol or NiTi], optionally nickel titanium wire. In some embodiments, the at least one resilient arm, for example at least one securing means, is made of titanium or another suitable metal or polymer. The at least one resilient arm, for example at least one securing means, can be constrained, by releasable constraining means, and the at least one resilient arm, for example at least one securing means, held parallel to the fixation element or pivotable clamp 18 in a first configuration on the outside surface of the pivotable clamp 18 that engages the native leaflet, for example the first arm or first rigid arm of the pivotable clamp 18. Once the final position has been achieved the at least one resilient arm, for example at least one securing means, can be unconstrained, from the releasable constraining means, and due to the superelastic nature of the material it will spring into a second configuration. In at least one embodiment, in the second configuration of the resilient arm, for example at least one securing means; part of the fixation element, such as the pivotable clamp 18 , forms a "T" shape on the ventricular surface of the native leaflet. The resilient horizontal element of the "T" [the at least one resilient arm, for example at least one securing means] lies on the superior surface of the pivotable clamp 18. The bottom of the "T" is the point of the pivot of the pivotable clamp 18. In some embodiments the horizontal element of the "T" [the at least one resilient arm, for example at least one securing means] is curved inwards applying a clamping pressure on the ventricular surface of the native leaflet, thus increasing the clamping zone of the pivotable clamp 18. In some embodiments the at least one resilient arm, for example at least one securing means, can be covered in a porous polymeric material surface cover for encouraging fibrotic tissue growth, or overgrowth, this will aid the long-term fixation of the device. Furthermore, the outside surface of the pivotable clamp 18 can be covered with a porous polymeric material surface cover for encouraging fibrotic tissue growth or overgrowth, aiding fixation and eliminating the need for anti-coagulation therapy associated with the exposure of these elements.

In some embodiments the pivotable clamp 18 may have a "ball nose" tip, this feature will reduce the damage to the myocardium as the open clip is manipulated into position.

In some embodiments, the guide 14 is integrated into the stented valve frame, for example an expandable stent scaffold 20.

In some embodiments, the guide 14 comprises a track. In such embodiments, the connector 12 communicates with the track.

In some embodiments, the guide 14 comprises a slot 24, or a channel, for example a slot 24, or channel, machined into the expandable stent scaffold 20. In some embodiments, the connector 12 comprises a projection 22 that communicates with the channel, or grove. In some such embodiments, the connector 12 comprises a projection 22 that traverses the slot 24 at the base of the projection 22, the end of the projection 22 being wider than the slot 24.

In some embodiments, the guide 14 comprises a tube, or tube lumen, optionally a slit tube or slit tube lumen. In such embodiments, the connector 12 communicates with the lumen of the guide 14, optionally the tube lumen. In some embodiments, the tube lumen has a non-circular cross-section.

In some embodiments, the connector 12 cannot rotate relative to the guide 14. In some embodiments, the pivotable clamp 18 cannot rotate relative to the guide 14. In some embodiments, the fixation element cannot rotate relative to the guide 14.

In some embodiments, rotation of the fixation element is eliminated and the translation of the fixation element is well controlled along a fixed path. In some embodiments, the fixed path is defined by the guide 14.

In some embodiments, the control rod 16 is capable of transmitting torque from the proximal end to the distal end. In some embodiments, the rotation of the proximal end of the control rod 16 interacts with the pivotable clamp 18 via a screw mechanism at or adjacent to the distal end of the control rod 16. In some embodiments, the control rod 16 is capable of transmitting contact force from the proximal end of the control rod 16 to the distal end of the control rod 16. In some embodiments, reciprocal movement of the proximal end of the control rod 16 parallel to the longitudinal axis of the control rod 16 leads to reciprocal movement of the connector 12 relative to the guide 14.

In some embodiments, the control rod 16 comprises resilient material, or the control rod 16 is formed from resilient material, or the control rod 16 is formed from a material flexible along the longitudinal axis of the control rod 16. In some embodiments, the control rod 16 has axial flexibility.

In some embodiments, the control rod 16 comprises at least one depression in the surface of the control rod 16, for example at least one groove. In some embodiments, the at least one groove is substantially perpendicular to the longitudinal axis of the control rod 16, such that the control rod 16 is flexible along the longitudinal axis of the control rod 16. In such embodiments, the control rod 16 formed from a stiff material, optionally metal, for example stainless steel, may be flexible along the longitudinal axis of the control rod 16.

In some embodiments, the at least one groove is manufacturable by cutting, optionally by laser cutting.

In some embodiments, the control rod 16 comprises a metal, for example a metal alloy such as a metal alloy of nickel and titanium, or a steel alloy, optionally stainless steel. In some embodiments, the control rod 16 comprises a metal alloy of cobalt and chromium.

In some embodiments, the fixation element further comprises a string, which may be reversibly connectable to the pivotable clamp 18. In some embodiments, the string is reversibly connectable to the control rod 16, optionally the proximal end of the control rod 16.

In some embodiments, the fixation element further comprises a controller 36, which the control rod 16 may interact with, optionally at the proximal end of the control rod 16. In some embodiments, the controller 36 is operable to actuate the pivotable clamp 18 via the control rod 16, for example to close and/or open the pivotable clamp 18 via the control rod 16.

In some embodiments, the controller is operable to move the sleeve 26 relative to the control rod 16.

In some embodiments, the controller comprises a rod control part and a sleeve control part. For example, the rod control part may be connected to the sleeve control part.

In some embodiments, the rod control part may act on the control rod 16. For example, the rod control part may be operable to rotate the control rod 16 about the longitudinal axis of the control rod 16.

In some embodiments, the sleeve control part may act on the sleeve 26. For example, the sleeve control part may be operable to rotate the sleeve 26 about the longitudinal axis of the control rod 16. In some embodiments, the sleeve control part is operable to move the sleeve 26 relative to the control rod 16, or, the sleeve control part is operable to reciprocally move the sleeve 26 relative to the control rod 16.

In some embodiments, the controller further comprises a spacer 46; or the controller may further comprise a spacer 46. In some embodiments, the rod control part may be connected to the sleeve control part by the spacer 46. In some embodiments, the spacer 46 may be displaced such that the rod control part may contact the sleeve control part.

In some embodiments, the fixation element further comprises a string, which may be reversibly connectable to the pivotable clamp 18, and/or reversibly connectable to the control rod 16, optionally at the proximal end of the control rod 16.

In some embodiments, the string may interact with the controller 36. In some embodiments, the controller 36 is operable to actuate the pivotable clamp 18 via the string; for example to close and/or open the pivotable clamp 18 via the string.

In some embodiments, the string and control rod 16 may interact with the controller 36. In some embodiments, the controller 36 is operable to actuate the pivotable clamp 18 via the string and control rod 16; for example to open and/or close the pivotable clamp 18 via the sting and control rod 16.

In some embodiments, the fixation element further comprises a sleeve 26 having a sleeve lumen. In some embodiments, the sleeve 26 is arranged around the control rod 16. The Control rod 16 may pass through the sleeve lumen, or through a first section of the length of the sleeve lumen.

In some embodiments, the string passes through the sleeve lumen, or through a second section of the length of the sleeve lumen.

In some embodiments, the sleeve 26 extends along the length of the control rod 16, from the proximal end of the control rod 16 to the distal end of the control rod 16. Alternatively, the sleeve 26 may extend along a section of the length of the control rod 16. In some embodiments, the sleeve 26 is moveable relative to the control rod 16.

In some embodiments, the sleeve 26 covers the distal end of the control rod 16, or the sleeve 26 is moveable (or reversibly movable) to cover the distal end of the control rod 16. In some embodiments, the sleeve 26 does not cover the distal end of the control rod 16, or the sleeve 26 is moveable (or reversibly movable) to uncover at least part of the distal end of the control rod 16. In some embodiments, the sleeve 26 is reversibly movable to cover, or uncover, at least part of the distal end of the control rod 16 such that a reversible connection between the distal end of the control rod 16 and the pivotable clamp 18 may be reversibly covered by the sleeve 26.

In some embodiments, the sleeve 26 supports the connection between the distal end of the control rod 16 and the pivotable clamp 18 whenever the sleeve 26 is covering the connection between the distal end of the control rod 16 and the pivotable clamp 18; such that moving the sleeve 26 to uncover the connection between the distal end of the control rod 16 and the pivotable clamp 18 may lead to disconnection of the distal end of the control rod 16 from the pivotable clamp 18.

In some embodiments the posterior side of the device can be modified, to ensure that a short native posterior leaflet can be captured. To this end the guide 14 can be located further superiorly on the main valve frame, this will allow transition of the fixation element, and/or pivotable clamp 18, to an appropriate height, ensuring capture of the native posterior leaflet. In some embodiments a longer fixation element, and/or pivotable clamp 18, can be used to secure the stented valve to the native leaflets, the fixation element may need to be longer than the anterior element.

The prosthetic valve according to some embodiments of the invention, comprising for example: a stented valve, such as an expandable stent scaffold 20, at least one coupling, at least one fixation element, and at least one control rod 16, are loaded into the delivery tube from the distal end of the delivery tube, this can be achieved either through adjunctive crimping tools or a funnel arrangement. The fixation elements are loaded closest to the distal end of the delivery tube, followed in series by the expandable stent scaffold 20, which occupies the next section of the delivery tube. The delivery tube can then be navigated to the target site either through the venous system and a transeptal puncture, or via a minimally invasive transatrial entrance to the heart.

Once the cardiologist/operator is satisfied with the delivery system location he/she can initiate the unsheathing of the fixation elements and the stent frame. In this instance the fixation elements and the stented valve remain in a static position and the outside tube is withdrawn relative to the implantable elements. Firstly, the outer sheath is withdrawn exposing the fixation elements in the left ventricle, this is followed by exposing the stented valve, this unsheathing should occur at the ideal landing zone for the stented valve e.g. the positioning protocol is designed that the annulus of the stented valve would closely align with the annulus of the native mitral valve (the ideal position is that the implant's annulus is 1-6 mm lower than the annulus at the A2 or P2 pivot points of the native leaflets). Or similarly if the technology is transferred to the tricuspid valve, the stented valve's annulus would be positioned at the same height as the native tricuspid annulus (or slightly lower, or slightly higher).

The next step in the process involves the opening of one of the fixation elements. This is conducted via mechanisms associated with the control rod 16 (In some embodiments, the control rods 16 are attached to the pivotable clamps 18 and lie in parallel with the pivotable clamps 18 and stented valve in the delivery system tubing. The control rods 16 attach to the pivotable clamps 18 at their distal tips and at the proximal end they can interact with the controller 36. The controller 36 interaction allows the user to position the fixation elements and/or pivotable clamps 18 in the desired location and the controller 36 also allows the pivotable clamps 18 to be opened or closed independently of moving the stented valve). The controller 36 and the handle may be separate, or these elements may interact or be connected.

In the open position, the fixation element can be manoeuvred back capturing the native leaflet and the stent frame in between the two arms or multiple arms of the pivotable clamp 18. Subsequently the fixation element is pulled (or pushed if transapical approach) to capture the native valve and stented valve. Once the fixation element has been navigated into the ideal position and the operator is satisfied with the fixation element's position he/she can close the pivotable clamp 18 via the control rod 16 mechanism in the controller 36. A similar procedure is initiated with the posterior fixation element: the pivotable clamp 18 is opened via the control rod 16; subsequently the fixation element is pulled/pushed/moved into a position that the "V' of the pivotable clamp 18 captures the native leaflet and the stented valve; if the operator is satisfied with the posterior fixation element's position they can close the fixation element via the control rod's 16 mechanism. In other embodiments additional fixation elements and/or pivotable clamps 18 could be utilised at other points of the circumference of the stented valve. At this point the fixation elements could either be released from the control rods 16 or remain attached. At this point the stented valve can be released from its release mechanism in the delivery catheter. (At the proximal end of the stented valve a release mechanism is coupled to the delivery system; the release mechanism allows the stented valve to be released in a controlled manner at the ideal anatomical landing zone). The stent release can be completed by withdrawing an internal mechanism that is located within the delivery system (this mechanism holds eyelets on the stented valve in close apposition to receiving features on a central core). The central core can be hollow in nature, allowing the use of guide 14wires in the system. Once the eyelets have been released the stented valve is no longer attached to the delivery system. However the fixation elements are still attached and need to be removed from the delivery system. For example, the at least one control rod 16 is still attached to the at least one respective pivotable clamp 18. This disconnection is achieved in one of a number of ways. For example the fixation elements can be removed by finalising closure of the fixation elements. E.g. the control rods 16 can be twisted further engaging a locking feature on the fixation elements. Once this feature has been engaged the pivotable clamps 18 can no longer be opened. However, this allows the control rods 16 to be unscrewed and removed from the system leaving pivotable clamps 18 closed and in their locked configuration; locking the stented valve to the native valve.

Further embodiments of this system include three fixation elements. In some embodiments, the location of the fixation elements and associated guides 14 mimic the locations of commissure posts on a surgical biological valve implant. For example the Edwards Perimount mitral valve replacement has three commissure posts, and additionally other geometric features including: a structural ring and an atrial flange. In these embodiments of the invention the fixation elements, guides 14, and commissures posts are co-localised or share the same geometrical location. Such embodiments are a first step towards achieving a "surgical valve type" geometry in a minimally invasive configuration. The second design features in this embodiment rely on minimising the length of the longest dimension of the stented valve, to result in a geometry that closely resembles a known surgical biological mitral valve replacement. For example, the fixation elements and guides 14 and commisures posts represent one element of known surgical valves, while one short row of stent cells or another expandable "ring" configuration represents the ring structural support, while the atrial flange represents the other key element of a known surgical biological mitral valve replacement. Combining the fixation method and the above geometrical features, a geometry that closely aligns with the surgical biological valve geometry can be achieved in the present invention, allowing for minimally invasive, beating heart delivery. The risks associated with surgical valve geometries are reasonably well known, while the risks associated with stented mitral valve geometries are not well understood. Therefore, the adoption of a minimally invasive AV valve that mimics surgical valve bioprosthesis geometry potentially could reduce the risks associated with minimally invasive mitral or tricuspid valve replacement.

A second aspect of the present invention comprises a coupling for a stented valve and the fixation element according to a first aspect of the present invention, the coupling comprising: (a) a connector 12 adapted to receive the fixation element, and (b) a guide 14 adapted to receive the stented valve; wherein the connector 12 is reciprocally movable relative to the guide 14.

The connector 12 may be slidable relative to the guide 14. In some embodiments, the connector 12 is reciprocally slidable relative to the guide 14. The connector 12 may be in communication with the guide 14. For example, the connector 12 may be in moveable communication with the guide 14, or the connector 12 may be in slidable communication with the guide 14.

In some embodiments, the connector 12 is insertable into the guide 14; and/or the connector 12 is retractable from the guide 14. In some embodiments, the connector 12 and guide 14 have a male-female interaction.

In some embodiments, the guide 14 is a pocket. The connector 12 may be insertable into the pocket and/or retractable from the pocket. For example, the connector 12 and pocket may have a male-female interaction.

In some embodiments, the guide 14 is a pocket in the stented valve.

In some embodiments, the guide 14 has a first end and a second end; whereby the connector 12 is reciprocally movable, or slidable, or reciprocally slidable between the first end and second end of the guide 14.

The connector 12 may be receivable within the guide 14. In some embodiments, the connector 12 is reversibly receivable within the guide 14. Alternatively, the connector 12 may be irreversibly receivable within the guide 14. In some embodiments, the connector 12 comprises a projection 22 receivable, or reversibly receivable within the guide 14. Alternatively, the connector 12 may comprise a projection 22 irreversibly receivable within the guide 14. In some embodiments, the guide 14 comprises a slot 24, and the connector 12 comprises a projection 22 receivable within the slot 24. In some embodiments, the projection 22 comprises a stop 23 to inhibit the passage of the connector 12 through the slot 24. Alternatively, in some embodiments, the guide 14 comprises a tube having a tube lumen, and the connector 12 is receivable within the guide 14. In some embodiments, the connector 12 is locatable, or reversibly locatable, within the guide 14. Alternatively, the connector 12 may be irreversibly locatable within the guide 14. In some embodiments, the connector 12 is concentrically locatable within the guide 14. For example, the connector 12 may be concentrically reversibly locatable within the guide 14, or tube lumen; or the connector 12 may be concentrically irreversibly locatable within the guide 14, or tube lumen. The connector 12 may be coaxially locatable within the tube lumen. For example, the connector 12 may be coaxially reversibly locatable within the tube lumen; or the connector 12 may be coaxially irreversibly locatable within the tube lumen.

In some embodiments, the connector 12 is not rotatable within the guide 14. For example, at least one, or both, of the tube lumen and the connector 12 may have a non-circular cross-section.

Also disclosed is a method of manufacture of a coupling as disclosed herein.

A first aspect of the present invention comprises a fixation element for fastening a stented valve to a native heart valve, the fixation element comprising: (a) a control rod 16 comprising: (i) a proximal end, connected to (ii) a distal end reversibly connectable to (b) a pivotable clamp 18 comprising (i) at least one arm, wherein the at least one arm comprises a hand, wherein the at least one arm and the hand are located on opposing sides of the pivot of the pivotable clamp; and (ii) an actuator operable to displace the pivotable clamp between an open position and a closed position by operation of the proximal end of the control rod 16.

In some embodiments, the distal end of the control rod 16 is reversibly connectable to the pivotable clamp 18 by an interference fit. For example, the distal end of the control rod 16 may comprise a key and the pivotable clamp 18 may comprise a socket. The key may be receivable within the socket. In some embodiments, the key is reversibly receivable within the socket, which may be by an interference fit. For example, the distal end of the control rod 16 may comprise a hex key and the pivotable clamp 18 may comprise a hex socket. Alternatively, in some embodiments the distal end of the control rod 16 comprises a threaded portion and the pivotable clamp 18 comprises a corresponding threaded socket. For example, the threaded portion may be receivable, or reversibly receivable, within the corresponding threaded socket.

The pivotable clamp 18 comprises an actuator to displace the pivotable clamp 18 between the open position and the closed position. For example, the actuator may comprise a reciprocally movable shaft, or reciprocally movable threaded shaft. In some embodiments, the actuator comprises a reciprocally movable threaded shaft 29 and a correspondingly threaded aperture 39. In some embodiments, the reciprocally movable shaft may act on the pivotable clamp 18.

In some embodiments, the pivotable clamp 18 comprises at least two arms. For example, the pivotable clamp 18 may comprise two arms, or the pivotable clamp 18 may comprise first and second arms. In some embodiments, the reciprocally movable shaft may act on at least one arm 28 of the pivotable clamp 18.

The at least one arm 28 of the pivotable clamp 18 comprises a hand. For example, the hand may be fixed to the at least one arm 28 of the pivotable clamp 18. The at least one arm 28 and the hand are located on opposing sides of the pivot of the pivotable clamp 18. In some embodiments, the reciprocally movable shaft may act on the hand of the pivotable clamp 18. For example, the reciprocally movable shaft may act on the hand of the pivotable clamp 18 to displace the at least one arm 28 between the open position and the closed position. In some embodiments, the reciprocally movable shaft may act on the hand of the pivotable clamp 18 to displace the at least one arm 28 about the pivot of the pivotable clamp 18.

The distal end of the control rod 16 may be reversibly connectable to the actuator by an interference fit. In some embodiments, the distal end of the control rod 16 comprises a key and the actuator comprises a socket. The key may be receivable within the socket. In some embodiments, the key is reversibly receivable within the socket, which may be by an interference fit. For example, the distal end of the control rod 16 may comprise a hex key and the actuator may comprise a hex socket. The hex key may be receivable, or reversibly receivable, within the hex socket. For example, the hex key may be reversibly receivable within the hex socket by an interference fit.

In some embodiments, the pivotable clamp 18 is displaceable between the closed position and a locked configuration; whereby operation of the proximal end of the control rod 16 does not displace the pivotable clamp 18 between the locked configuration and the open position.

In some embodiments, the pivotable clamp 18 is displaceable between the closed position and a locked configuration by operation of the proximal end of the control rod 16.

In some embodiments, the pivotable clamp 18 further comprises a lock that is operable to displace the pivotable clamp 18 into the locked configuration. For example, the actuator may act on the lock; for example the actuator may act on the lock to displace the pivotable clamp 18 between the closed position and the locked configuration.

In some embodiments, the lock further comprises at least one elastic member 38.

In some embodiments, the lock further comprises at least one elastic member 38 having an elastic limit and an elastic displacement zone. In some embodiments, the at least one elastic member 38 may be reversibly deformed by displacement of the pivotable clamp 18 from the open position to the closed position. If deformed beyond the elastic limit, the at least one elastic member 38 may be irreversibly deformed. In some embodiments, the locked configuration of the pivotable clamp 18 is the configuration wherein the at least one elastic member 38 is irreversibly deformed.

In some embodiments, the at least one elastic member 38 may be deformed by movement of the actuator, for example by movement of the reciprocally movable shaft. In some embodiments, the at least one elastic member 38 is connected to the hand of the pivotable clamp 18.

In some embodiments, the at least one elastic member 38 may be reversibly deformed from an original position to a deformed position.

In some embodiments, in the deformed position the at least one elastic member 38 is in contact with the exterior surface of the pivotable clamp 18; optionally in the deformed position the at least one elastic member 38 is in slidable contact with the exterior surface of the pivotable clamp 18.

In some embodiments, in the deformed position the at least one elastic member 38 is in contact with the hand of the pivotable clamp 18; optionally in the deformed position the at least one elastic member 38 is in slidable contact with the hand of the pivotable clamp 18.

In some embodiments, the at least one elastic member 38 is in the deformed position in the open position of the pivotable clamp 18. In some embodiments, the at least one elastic member 38 is in the deformed position in the closed position of the pivotable clamp 18. In some embodiments, the at least one elastic member 38 is in the original position in the locked configuration of the pivotable clamp 18. In some embodiments the at least one elastic member 38 is moved by the elasticity of the at least one elastic member 38 to the original position in the locked configuration of the pivotable clamp 18.

In some embodiments, the at least one elastic member 38 prevents the pivotable clamp 18 from moving from the locked configuration of the pivotable clamp 18 to the open position of the pivotable clamp 18.

In some embodiments, the actuator is detachable, optionally reversibly detachable, from the pivotable clamp 18. In some embodiments, the reciprocally movable shaft is detachable, optionally reversibly detachable, from the pivotable clamp 18; optionally the reciprocally movable threaded shaft 29 is detachable, optionally reversibly detachable, from the pivotable clamp 18.

In some embodiments, the at least one elastic member 38 prevents the pivotable clamp 18 from moving from the locked configuration of the pivotable clamp 18 to the open position of the pivotable clamp 18, in the detached configuration of the actuator. In some embodiments, the at least one elastic member 38 prevents the pivotable clamp 18 from moving from the locked configuration of the pivotable clamp 18 to the open position of the pivotable clamp 18, in the detached configuration of the reciprocally movable shaft. In some embodiments, the at least one elastic member 38 prevents the pivotable clamp 18 from moving from the locked configuration of the pivotable clamp 18 to the open position of the pivotable clamp 18, in the detached configuration of the reciprocally movable threaded shaft 29.

In some embodiments, the control rod 16 comprises the actuator, or reciprocally movable shaft, or reciprocally movable threaded shaft 29. In some embodiments, the control rod 16 comprises the actuator, or reciprocally movable shaft, or reciprocally movable threaded shaft 29. In some embodiments, the control rod 16 comprises the actuator, or reciprocally movable shaft, or reciprocally movable threaded shaft 29. In some embodiments, the distal end of the control rod 16 comprises the actuator, or reciprocally movable shaft, or reciprocally movable threaded shaft 29.

In some embodiments, when the pivotable clamp 18 is assembled, the at least one elastic member 38 is elastically deformed from the original position to a deformed position that, in some embodiments, allows them to slide on the exterior surface of the pivotable clamp 18. In the majority of the opening and closing stroke of the pivotable clamp 18, for example the open position and closed position, the at least one elastic member 38 may slide on the outside of the pivotable clamp 18, for example the at least one elastic member 38 may be in slidable contact with the exterior surface of the pivotable clamp 18. However, once the pivotable clamp 18 is moved into the locked configuration, the at least one elastic member 38 may "bounce" back into the original position not on the side of the pivotable clamp 18, for example the at least one elastic member 38 may be moved by the elasticity of the at least one elastic member 38 into the original position, in the locked configuration of the pivotable clamp 18. In the original position, the at least one elastic member 38 does not allow any further opening of the pivotable clamp 18.

In some embodiments, the arrangement of the lock could be different, for example the at least one elastic member 38 could operate on the hand rather than the actuator, or reciprocally movable shaft, or reciprocally movable threaded shaft 29.

In some embodiments, the reciprocally moveable shaft may be released, or detached, from the pivotable clamp 18; for example by unscrewing the reciprocally movable threaded shaft 29 from the correspondingly threaded aperture. In some embodiments, this would not allow the locked configuration of the pivotable clamp 18 to move to the open position of the pivotable clamp 18, as in some embodiments the pivotable clamp 18 does not require the reciprocally moveable shaft or the actuator to keep the pivotable clamp 18 in the locked configuration.

In some embodiments, the at least one elastic member 38 does not contact the actuator in the open position of the pivotable clamp 18; and the at least one elastic member 38 may be substantially parallel to the actuator in the open position of the pivotable clamp 18. In some embodiments, the at least one elastic member 38 contacts the actuator in the locked configuration of the pivotable clamp 18.

In some embodiments, the at least one elastic member 38 does not contact the reciprocally movable shaft in the open position of the pivotable clamp 18; and the at least one elastic member 38 may be substantially parallel to the reciprocally movable shaft in the open position of the pivotable clamp 18. In some embodiments, the at least one elastic member 38 contacts the reciprocally movable shaft in the locked configuration of the pivotable clamp 18.

In some embodiments, the at least one elastic member 38 does not contact the reciprocally movable threaded shaft 29 in the open position of the pivotable clamp 18; and the at least one elastic member 38 may be substantially parallel to the reciprocally movable threaded shaft 29 in the open position of the pivotable clamp 18. In some embodiments, the at least one elastic member 38 contacts the reciprocally movable threaded shaft 29 in the locked configuration of the pivotable clamp 18.

In some embodiments, the pivotable clamp 18 comprises an offset hinge. For example, an offset hinge of the pivotable clamp 18 may prevent the pivotable clamp 18 from obstructing movement of the connector 12 between the first end and second end of the guide 14.

In some embodiments, the pivotable clamp 18 comprises first and second arms connected by a pivot. In some embodiments, the pivotable clamp 18 further comprises first and second sub-arms connected by a sub-pivot. For example, the first sub-arm may be mountable to the first arm and the second sub-arm may be mountable to the second arm. In some embodiments, the first sub-arm is fixedly mountable to the first arm and the second sub-arm is pivotably mountable to the second arm.

In some embodiments, at least one arm 28 of the pivotable clamp 18 further comprises a ball nose tip.

In some embodiments, at least one arm 28 of the pivotable clamp 18 further comprises securing means. The securing means may be operable between a contracted state and an expanded state. In some embodiments, in the contracted state the securing means are substantially coaxial with the longitudinal axis of the at least one arm 28. In some embodiments, in the expanded state, the securing means are substantially perpendicular to the longitudinal axis of the at least one arm 28. The securing means may be formed from a resilient material. For example, the securing means may be formed from a metal, or a metal alloy, or a metal alloy of nickel and titanium.

In some embodiments, the at least one securing means further comprises a surface cover for encouraging fibrotic tissue growth, the surface cover comprising: porous polymeric material. For example, the surface cover may comprise a material selected from the group comprising: porous polymeric plastic, polyester, and synthetic polyester. In some embodiments, the surface cover comprises a material selected from the group comprising: polyethylene terephthalate (PET), polyethylene terephthalate fabric (PET fabric), polytetrafluoroethylene (PTFE), and expanded polytetrafluoroethylene (ePTFE).

In some embodiments, operation of the proximal end of the control rod 16 actuates operation of the distal end of the control rod 16. In some embodiments, operation of the distal end of the control rod 16 actuates operation of the pivotable clamp 18. For example, operation of the key of the distal end of the control rod 16 may actuate operation of the socket of the pivotable clamp 18. In some embodiments, operation of socket of the pivotable clamp 18 actuates the actuator to displace the pivotable clamp 18 between the open position and the closed position. In some embodiments, operation of socket of the pivotable clamp 18 actuates the reciprocally movable threaded shaft 29 in the correspondingly threaded aperture 39. For example, operation of the reciprocally movable threaded shaft 29 in the correspondingly threaded aperture 39 may act on at least one arm 28 of the pivotable clamp 18, or may act on the hand of the at least one arm 28 of the pivotable clamp 18. In some embodiments, operation of the reciprocally movable threaded shaft 29 in the correspondingly threaded aperture 39 may act on the hand of the pivotable clamp 18 to displace the at least one arm 28 between the open position and the closed position, for example about the pivot of the pivotable clamp 18.

Also disclosed is a method of manufacture of a fixation element as described herein.

A third aspect of the present invention comprises a prosthetic valve comprising a stented valve and at least one fixation element according to a first aspect of the [resent invention, wherein the stented valve and the at least one fixation element are each received by at least one coupling.

In some embodiments, the prosthetic valve comprises a stented valve, and at least one fixation element according to a first aspect of the [resent invention, wherein the stented valve and the at least one fixation element are each received by at least one coupling according to the second aspect of the present invention.

The stented valve can comprise: (a) at least one coupling, each coupling comprising: (i) a connector 12 adapted to receive the fixation element, and (ii) a guide 14 adapted to receive the stented valve; wherein the connector 12 is reciprocally movable relative to the guide 14; and (b) at least one respective fixation element. For example, the stented valve may comprise: (a) two, three or a plurality of couplings, each coupling comprising: (i) a connector 12 adapted to receive the fixation element, and (ii) a guide 14 adapted to receive the stented valve; wherein the connector 12 is reciprocally movable relative to the guide 14; and (b) two, three, or a plurality of respective fixation elements.

The fixation element in the third aspect of the present invention is a fixation element according to the second aspect of the present invention.

In some embodiments, the stented valve comprises: (a) a coupling comprising: (i) a connector 12 adapted to receive the fixation element, and (ii) a guide 14 adapted to receive the stented valve; wherein the connector 12 is reciprocally movable relative to the guide 14; and (b) a fixation element comprising: (i) a control rod 16 comprising: a proximal end, connected to a distal end reversibly connectable to (ii) a pivotable clamp 18 comprising (i) at least one arm, wherein the at least one arm comprises a hand, wherein the at least one arm and the hand are located on opposing sides of the pivot of the pivotable clamp; and (ii) an actuator operable to displace the pivotable clamp between an open position and a closed position by operation of the proximal end of the control rod 16.

In some embodiments, the longitudinal axis of the coupling is substantially coaxial with the longitudinal axis of the stented valve. For example, the longitudinal axis of the coupling, the longitudinal axis of the stented valve, and the longitudinal axis of the fixation element may be each substantially coaxial.

In some embodiments, the at least one coupling and the at least one fixation element are co-localised.

In some embodiments, the stented valve further comprises at least one commissures post. For example, the stented valve may further comprise two, three, or a plurality of commissures posts.

In some embodiments, the at least one commissures post and the at least one coupling are co-localised. In some embodiments, the at least one commissures post and the at least one coupling and the at least one respective fixation element are co-localised. For example, the at least one commissures post may be connected to the at least one coupling, or the at least one commissures post is connected to the at least one coupling via the respective guide 14.

In some embodiments, the stented valve comprises an expandable stent scaffold 20. In some embodiments, the guide 14 is adapted to receive the expandable stent scaffold 20. For example, the guide 14 may be adapted to receive the expandable stent scaffold 20 such that the longitudinal axis of the guide 14 is substantially coaxial with the longitudinal axis of the expandable stent scaffold 20. In some embodiments, the longitudinal axis of the guide 14, the longitudinal axis of the expandable stent scaffold 20, and the longitudinal axis of the fixation element are each substantially coaxial.

In some embodiments, the expandable stent scaffold 20 is formed from a resilient material. For example, the at least one ribbon may be formed from a resilient material. In some embodiments, the expandable stent scaffold 20 is formed from a metal. For example, the expandable stent scaffold 20 may be formed from a metal alloy, or a metal alloy of nickel and titanium, or a steel alloy, or stainless steel, or a metal alloy of cobalt and chromium.

In some embodiments, the expandable stent scaffold 20 is formed from a polymer material. For example, the expandable stent scaffold 20 may comprise material selected from the group comprising polymeric plastic material, organic thermoplastic polymer, polyaryletherketone (PAEK), polyether ether ketone (PEEK), synthetic polyester, and polytetrafluoroethylene (PTFE).

In some embodiments, the lattice comprises: at least one hinge at a ribbon intersection.

In some embodiments, the expandable stent scaffold 20 has a funnel shape. For example, the expandable stent scaffold 20 may have a funnel shape whereby the diameter of a first open end of the expandable stent scaffold 20 is greater than the diameter of a second open end of the expandable stent scaffold 20.

In some embodiments, the first open end of the expandable stent scaffold 20 defines the stent perimeter. In some embodiments, the at least one coupling defines the coupling perimeter, whereby the at least one coupling is contained within the coupling perimeter. In some embodiments, the coupling perimeter is shorter than the stent perimeter. Alternatively, the coupling perimeter may be longer than the stent perimeter. Alternatively, the coupling perimeter may be the same length as the stent perimeter.

In some embodiments, the at least one fixation element defines the fixation element perimeter, whereby the at least one fixation element is contained within the fixation element perimeter. In some embodiments, the fixation element perimeter is shorter than the stent perimeter. Alternatively, the fixation element perimeter may be longer than the stent perimeter. Alternatively, the fixation element perimeter may be the same length as the stent perimeter.

In some embodiments, the expandable stent scaffold 20 further comprises at least one eyelet connected to the lattice, whereby the at least one eyelet is adjacent to at least one open end of the expandable stent scaffold 20.

In some embodiments, the expandable stent scaffold 20 further comprises: a skirt. For example, the skirt may be inside, outside, or both inside and outside of the expandable stent scaffold 20 frame. In some embodiments, the skirt is integrally formed in the expandable stent scaffold 20. In some embodiments, the skirt is attached to the expandable stent scaffold 20. For example, the skirt may be attached to the expandable stent scaffold 20 by an adhesive selected from the group comprising: glue, fixative, gum, cement, bonding, binder, and sealant. In some embodiments, the skirt is attached to the expandable stent scaffold 20 by attachment means selected from the group comprising: stitching, sewing, and suturing. In some embodiments, the skirt is attached to the expandable stent scaffold 20 by attachment means selected from the group comprising welding, heat bonding, brazing, and soldering.

The longest dimension of the guide 14 may be longer than the longest dimension of the stented valve. Alternatively, in some embodiments, the longest dimension of the guide 14 is substantially the same length as the longest dimension of the stented valve, or shorter than the longest dimension of the stented valve. The longest dimension of the guide 14 may be about half the length of the longest dimension of the stented valve. In some embodiments, at least one terminal end of the guide 14, or each terminal end of the guide 14, extends beyond a terminal end of the stented valve. In some embodiments, the guide 14 extends beyond the stented valve further at one end of the guide 14 than at the other end of the guide 14. Minimising the length of the longest dimension of the stented valve, such that the longest dimension of the guide 14 is longer than the longest dimension of the stented valve results in a geometry that closely resembles known surgical biological mitral valve replacement.

In some embodiments, the stented valve does not act like a valve it acts like a valve spacer, or the stented valve is a valve spacer. The valve spacer is suitable to fill the regurgitant area of a diseased valve. The valve spacer comprises a 3D space that acts as a valve coaptation assist device. This valve spacer can be expandable via nitinol self expanding mesh technology or inflatable. In some embodiments, the valve spacer is connected to guides for the fixation elements to slide within, similar to the sliding relationship previously described in other embodiments. The guides could be, for example, located in the centre of the valve spacer, or connected to the exterior surface of the valve spacer. The valve spacer can be substantially cylindrical or it can be substantially conical in shape, with or without a truncated tip. The valve spacer could also have a cross section oval shaped or "kidney bean" shape, or "kidney bean" cross section. The external surface of the valve spacer could be manufactured from a soft material: polymeric natural or synthetic. This may limit damage to the functioning native leaflets. In this instance the valve spacer is "clipped" to the native valve via the fixation elements in a similar manner to the stented valve described earlier. The valve spacer does not replace the native valve it simply acts as a filler of the hole that would have existed in a diseased valve (i.e. in functional mitral valve disease). i.e. the valve spacer provides an additional surface for the native valve to coapt against, and the solid volume of the valve spacer fills the regurgitate area. This solves the problem of when the leaflets do not meet in functional mitral valve disease.

In some embodiments, the stented valve is a valve spacer.

In some embodiments, the valve spacer is suitable for filling the regurgitant area of a diseased valve, or to act like a valve coaption assist device.

In some embodiments, the valve spacer comprises a solid volume.

In some embodiments, the valve spacer is substantially cylindrical. In some embodiments, the valve spacer is substantially conical. In some embodiments, the valve spacer is substantially a truncated cone shape. In some embodiments, the valve spacer is substantially a frustrum shape.

In some embodiments, the valve spacer is substantially an ellipsoid shape. In some embodiments, the valve spacer is substantially a prolate spheroid shape.

In some embodiments, the valve spacer is substantially a kidney bean shape.

In some embodiments, the valve spacer has an oval cross-section. In some embodiments, the valve spacer has a kidney bean shaped cross-section.

In some embodiments, the valve spacer is expandable. In some embodiments, the valve spacer comprises nitinol. In some embodiments, the valve spacer comprises nitinol self-expanding mesh. In some embodiments, the valve spacer is inflatable.

In some embodiments, the external surface of the valve spacer comprises a soft material.

In some embodiments, the external surface of the valve spacer comprises a natural material.

In some embodiments, the external surface of the valve spacer comprises a synthetic material.

In some embodiments, the external surface of the valve spacer comprises a polymeric material.

In some embodiments, the external surface of the valve spacer comprises a material selected from the group comprising: plastic, polymeric plastic, polyester, polyethylene terephthalate (PET), polyethylene terephthalate fabric (PET fabric), polytetrafluoroethylene (PTFE), expanded polytetrafluoroethylene (ePTFE), organic thermoplastic polymer, polyaryletherketone (PAEK), and polyether ether ketone (PEEK).

Also disclosed is a method of manufacture of a prosthetic valve as described herein.

A method for repairing or replacing a dysfunctional heart valve using a prosthetic valve according to the third aspect of the present invention can comprise the steps of:
(i) providing a prosthetic valve as described herein;
(ii) moving the connector 12 of at least one coupling relative to the guide 14 of the same coupling;
(iii) displacing the pivotable clamp of at least one fixation element between the open position and the closed position by operation of the proximal end of the control rod 16 of the same fixation element; wherein the pivotable clamp captures a native leaflet;
(iv) disconnecting the distal end of the control rod 16 of at least one fixation element from the pivotable clamp of the same fixation element, and withdrawing the control rod 16 such that the prosthetic valve is anchored in an appropriate position.

A medical device can comprise: (a) a prosthetic valve according to the third aspect of the present invention, and (b) a delivery system for transluminally guiding the prosthetic valve and adapted to receive the stented valve.

In some embodiments, the delivery system comprises a delivery tube adapted to receive the prosthetic valve, and/or the stented valve. For example, the delivery tube may comprise a series of retractable telescopic tubes.

In some embodiments, the medical device further comprises a handle mechanism operable to control the expansion and release of the stented valve.

Also disclosed is a method of manufacture of a medical device as described herein.

A method for repairing or replacing a dysfunctional heart valve using a prosthetic valve according to the third aspect of the present invention can comprise the steps of:
(i) providing a medical device as described herein;
(ii) advancing at least part of the delivery system to the native valve;
(iii) unsheathing at least part of the prosthetic valve from the delivery system;
(iv) moving the connector 12 of at least one coupling relative to the guide 14 of the same coupling;
(v) displacing the pivotable clamp of at least one fixation element between the open position and the closed position by operation of the proximal end of the control rod 16 of the same fixation element; wherein the pivotable clamp captures a native leaflet;
(vi) disconnecting the distal end of the control rod 16 of at least one fixation element from the pivotable clamp of the same fixation element, and withdrawing the control rod 16;
(vii) withdrawing the delivery system;
such that the prosthetic valve is anchored in an appropriate position.

This invention addresses the problem of replacing a mitral valve in a minimally invasive manner. At present surgery is the gold standard, however over 50% of patients are denied surgery and currently have no interventional treatment. This invention is one solution that can provide an interventional treatment to these patients.

## Claims

1. A fixation element for fastening a stented valve to a native heart valve, the fixation element comprising:
(a) a control rod (16) comprising:
(i) a proximal end, connected to
(ii) a distal end reversibly connectable to
(b) a pivotable clamp (18) comprising
(i) at least one arm, wherein the at least one arm comprises a hand, wherein the at least one arm and the hand are located on opposing sides of the pivot of the pivotable clamp; and
(ii) an actuator operable to displace the pivotable clampbetween an open position and a closed position by operation of the proximal end of the control rod.

2. The fixation element according to Claim 1; wherein the actuator acts on the pivotable clamp to displace the pivotable clamp between the open position and the closed position.

3. The fixation element according to Claim 1 or 2; wherein the actuator acts on the hand of the pivotable clamp to displace the pivotable clamp between the open position and the closed position.

4. The fixation element according to any preceding claim, wherein the actuator is a reciprocally movable shaft (29).

5. The fixation element according to any preceding claim, wherein the actuator is a reciprocally movable threaded shaft and a correspondingly threaded aperture (39), whereby the distal end of the control rod is reversibly connectable to the reciprocally movable threaded shaft.

6. The fixation element according to Claim 6; wherein operation of the reciprocally movable threaded shaft in the correspondingly threaded aperture acts on the at least one arm (28) of the pivotable clamp.

7. The fixation element according to any one of Claims 1-3, wherein the actuator is a string connected to the pivotable clamp and reversibly connected to the control rod.

8. The fixation element according to Claim 7, wherein the actuator is a string connected to the hand of the pivotable clamp, and which acts on the hand of the pivotable clamp to displace the pivotable clamp between the open position and the closed position.

9. The fixation element according to any preceding claim, wherein the control rod is flexible.

10. A coupling for a stented valve and the fixation element according to any preceding claim, the coupling comprising: a connector (12) adapted to receive the fixation element, and a guide (14) adapted to receive the stented valve: wherein the connector is reciprocally movable relative to the guide.

11. A prosthetic valve comprising a stented valve, and at least one fixation element according to any preceding claim; wherein the stented valve and the at least one fixation element are each received by at least one coupling.

12. The prosthetic valve according to Claim 11; wherein the at least one coupling is the coupling according to Claim 10.

13. The prosthetic valve according to Claim 11 or 12; wherein the longitudinal axis of the coupling, the longitudinal axis of the stented valve, and the longitudinal axis of the fixation element are each substantially coaxial.

14. The prosthetic valve according to any one of Claims 11-13; wherein the stented valve is a valve spacer suitable to fill a hole in a diseased valve.

15. The prosthetic valve according to Claim 14; wherein the valve spacer is substantially cylindrical, substantially conical, a substantially truncated cone shape, or a substantially frustrum shape.

## Patentansprüche

1. Fixierelement zum Befestigen einer gestenteten Klappe an einer natürlichen Herzklappe, wobei das Fixierelement Folgendes umfasst:
(a) eine Steuerstange (16), umfassend:
(i) ein proximales Ende, verbunden mit
(ii) einem distalen Ende, das reversibel verbindbar ist mit
(b) einer schwenkbaren Klammer (18), umfassend
(i) mindestens einen Arm, wobei der mindestens eine Arm einen Griff umfasst, wobei sich der mindestens eine Arm und der Griff auf gegenüberliegenden Seiten des Drehpunkts der schwenkbaren Klammer befinden; und
(ii) einen Aktuator, der die schwenkbare Klammer durch Betätigung des proximalen Endes der Steuerstange zwischen einer offenen Stellung und einer geschlossenen Stellung verlagern kann.

2. Fixierelement nach Anspruch 1, wobei der Aktuator auf die schwenkbare Klammer einwirkt, um die schwenkbare Klammer zwischen der offenen Stellung und der geschlossenen Stellung zu verlagern.

3. Fixierelement nach Anspruch 1 oder 2, wobei der Aktuator auf den Griff der schwenkbaren Klammer einwirkt, um die schwenkbare Klammer zwischen der offenen Stellung und der geschlossenen Stellung zu verlagern.

4. Fixierelement nach einem der vorstehenden Ansprüche, wobei der Aktuator ein hin- und herbeweglicher Schaft (29) ist.

5. Fixierelement nach einem der vorstehenden Ansprüche, wobei der Aktuator ein hin- und herbeweglicher Gewindeschaft und eine entsprechende Gewindeöffnung (39) ist, wobei das distale Ende der Steuerstange reversibel mit dem hin- und herbeweglichen Gewindeschaft verbindbar ist.

6. Fixierelement nach Anspruch 6, wobei die Betätigung des hin- und herbeweglichen Gewindeschafts in der entsprechenden Gewindeöffnung auf den mindestens einen Arm (28) der schwenkbaren Klammer einwirkt.

7. Fixierelement nach einem der Ansprüche 1-3, wobei der Aktuator eine Schnur ist, die mit der schwenkbaren Klammer verbunden und reversibel mit der Steuerstange verbunden ist.

8. Fixierelement nach Anspruch 7, wobei der Aktuator eine Schnur ist, die mit dem Griff der schwenkbaren Klammer verbunden ist und auf den Griff der schwenkbaren Klammer einwirkt, um die schwenkbare Klammer zwischen der geöffneten Stellung und der geschlossenen Stellung zu verlagern.

9. Fixierelement nach einem der vorstehenden Ansprüche, wobei die Steuerstange flexibel ist.

10. Kupplung für eine gestentete Klappe und das Fixierelement nach einem der vorstehenden Ansprüche, wobei die Kupplung Folgendes umfasst: einen Verbinder (12), der zur Aufnahme des Fixierelements ausgelegt ist, und eine Führung (14), die zur Aufnahme der gestenteten Klappe ausgelegt ist: wobei der Verbinder in Bezug auf die Führung hin- und herbeweglich ist.

11. Künstliche Herzklappe, die eine gestentete Klappe und mindestens ein Fixierelement nach einem der vorstehenden Ansprüche umfasst, wobei die gestentete Klappe und das mindestens eine Fixierelement jeweils von mindestens einer Kupplung aufgenommen werden.

12. Künstliche Herzklappe nach Anspruch 11, wobei die mindestens eine Kupplung die Kupplung nach Anspruch 10 ist.

13. Künstliche Herzklappe nach Anspruch 11 oder 12, wobei die Längsachse der Kupplung, die Längsachse der gestenteten Klappe und die Längsachse des Fixierelements jeweils im Wesentlichen koaxial sind.

14. Künstliche Herzklappe nach einem der Ansprüche 11-13, wobei die gestentete Klappe ein Klappenabstandsstück ist, das zum Füllen eines Lochs in einer erkrankten Herzklappe geeignet ist.

15. Künstliche Herzklappe nach Anspruch 14, wobei das Klappenabstandsstück im Wesentlichen zylindrisch, im Wesentlichen konisch, im Wesentlichen abgeschrägt kegelstumpfförmig oder im Wesentlichen kegelstumpfförmig ist.

## Revendications

1. Élément de fixation destiné à fixer une valvule à stent à une valvule cardiaque native, l'élément de fixation comprenant :
(a) une tige de commande (16) comprenant :
(i) une extrémité proximale, reliée à
(ii) une extrémité distale pouvant être reliée de manière réversible à
(b) une pince pivotante (18) comprenant
(i) au moins un bras, dans lequel l'au moins un bras comprend une main, dans lequel l'au moins un bras et la main sont situés sur des côtés opposés du pivot de la pince pivotante ; et
(ii) un actionneur utilisable pour déplacer la pince pivotante entre une position ouverte et une position fermée par actionnement de l'extrémité proximale de la tige de commande.

2. Élément de fixation selon la revendication 1, dans lequel l'actionneur agit sur la pince pivotante pour déplacer la pince pivotante entre la position ouverte et la position fermée.

3. Élément de fixation selon la revendication 1 ou 2, dans lequel l'actionneur agit sur la main de la pince pivotante pour déplacer la pince pivotante entre la position ouverte et la position fermée.

4. Élément de fixation selon une quelconque revendication précédente, dans lequel l'actionneur est un arbre réciproquement mobile (29).

5. Élément de fixation selon une quelconque revendication précédente, dans lequel l'actionneur est un arbre fileté réciproquement mobile et une ouverture filetée en correspondance (39), moyennant quoi l'extrémité distale de la tige de commande peut être reliée de manière réversible à l'arbre fileté réciproquement mobile.

6. Élément de fixation selon la revendication 6, dans lequel le fonctionnement de l'arbre fileté réciproquement mobile dans l'ouverture filetée en correspondance agit sur l'au moins un bras (28) de la pince pivotante.

7. Élément de fixation selon l'une quelconque des revendications 1 à 3, dans lequel l'actionneur est une corde reliée à la pince pivotante et reliée de manière réversible à la tige de commande.

8. Élément de fixation selon la revendication 7, dans lequel l'actionneur est une corde reliée à la main de la pince pivotante, et qui agit sur la main de la pince pivotante pour déplacer la pince pivotante entre la position ouverte et la position fermée.

9. Élément de fixation selon une quelconque revendication précédente, dans lequel la tige de commande est flexible.

10. Couplage pour une valvule à stent et élément de fixation selon une quelconque revendication précédente, le couplage comprenant : un connecteur (12) adapté pour recevoir l'élément de fixation, et un guide (14) adapté pour recevoir la valvule à stent, dans lequel le connecteur est mutuellement mobile par rapport au guide.

11. Valvule prothétique comprenant une valvule à stent et au moins un élément de fixation selon une quelconque revendication précédente, dans laquelle la valvule à stent et l'au moins un élément de fixation sont chacun reçus par au moins un couplage.

12. Valvule prothétique selon la revendication 11, dans laquelle l'au moins un couplage est le couplage selon la revendication 10.

13. Valvule prothétique selon la revendication 11 ou 12, dans laquelle l'axe longitudinal du couplage, l'axe longitudinal de la valvule à stent et l'axe longitudinal de l'élément de fixation sont chacun sensiblement coaxiaux.

14. Valvule prothétique selon l'une quelconque des revendications 11 à 13, dans laquelle la valvule à stent est un dispositif d'espacement de valvule approprié pour remplir un orifice dans une valvule malade.

15. Valvule prothétique selon la revendication 14, dans laquelle le dispositif d'espacement de valvule est sensiblement cylindrique, sensiblement conique, sensiblement en forme de cône tronqué ou sensiblement en forme de tronc.
